# EUROPEAN PATENT SPECIFICATION

(11) **EP 3 849 573 B1**
(45) Date of publication and mention of the grant of the patent: **29.10.2025**
(21) Application number: 19859526.6
(22) Date of filing: 13.09.2019
(51) Int. Cl.: A61K 35/74, A61K 8/99, A61K 9/06, A61P 17/10

(54) **BACTERIOTHERAPY AGAINST PROPRIONIBACTERIUM ACNES FOR THE TREATMENT OF ACNE**
BAKTERIENTHERAPIE GEGEN PROPRIONIBACTERIUM ACNES ZUR BEHANDLUNG VON AKNE
BACTÉRIOTHÉRAPIE CONTRE PROPRIONIBACTERIUM ACNES POUR LE TRAITEMENT DE L'ACNÉ

(30) Priority: 13.09.2018 US 201862730999 P
(43) Date of publication of application: 21.07.2021
(73) Proprietor: The Regents of The University of California, Oakland, CA 94607 (US)
(72) Inventor: GALLO, Richard, L., San Diego, CA 92117 (US); NAKATSUJI, Teruaki, San Diego, CA 92126 (US); O'NEILL, Alan, O., San Diego, CA 92109 (US)
(74) Representative: Gill Jennings & Every LLP
(86) International application number: PCT/US2019/051156
(87) International publication number: WO 2020/056359

(56) References cited:
- WO-A1-2015/097061
- WO-A1-2015/106175
- WO-A2-2016/179440
- ANNA L. COGEN ET AL: "Selective Antimicrobial Action Is Provided by Phenol-Soluble Modulins Derived from Staphylococcus epidermidis, a Normal Resident of the Skin", JOURNAL OF INVESTIGATIVE DERMATOLOGY, vol. 130, no. 1, 1 January 2010 (2010-01-01), NL, pages 192 - 200, XP055531093, ISSN: 0022-202X, DOI: 10.1038/jid.2009.243
- KUMAR ROHIT ET AL: "Genome Analysis of Staphylococcus capitis TE8 Reveals Repertoire of Antimicrobial Peptides and Adaptation Strategies for Growth on Human Skin", SCIENTIFIC REPORTS, vol. 7, no. 1, 5 September 2017 (2017-09-05), pages 1 - 10, XP055811482, Retrieved from the Internet <URL:https://www.ncbi.nlm.nih.gov/pmc/articles/PMC5585272/pdf/41598_2017_Article_11020.pdf> DOI: 10.1038/s41598-017-11020-7
- "The Infectious Etiology of Chronic Diseases: Defining the Relationship, Enhancing the Research, and Mitigating the Effects", 1 January 2004, Washington (DC), ISBN: 978-0-309-52672-2, article AJAY BHATIA , JEAN-FRANCOISE MAISONNEUVE , DAVIS H PERSING : "Propionibacterium Acnes And Chronic Diseases", pages: 74 - 80, XP008180264
- SHU LI, HUI HUANG, XIANCAI RAO, WEI CHEN, ZHENGQING WANG & XIAOMEI HU: "Phenol-Soluble Modulins: Novel Virulence-Associated Peptides of Staphylococci", FUTURE MICROBIOLOGY, vol. 9, no. 2, 3 December 2013 (2013-12-03), pages 203 - 216, XP009526255, ISSN: 1746-0913, DOI: 10.2217/fmb.13.153

## Description

### TECHNICAL FIELD

The invention relates to compositions and medical treatments for dermatological diseases and disorders and to composition and formulations to treat acne.

### BACKGROUND

The human skin harbors diverse microbial communities which constitute important element of innate immune barrier to defend the host from pathogens.

WO2015/106175 A1 discloses a topical probiotic composition for producing or maintaining skin microbiome balance, comprising commensal skin bacteria and their fermentation extracts. Cogen et al. "Selective antimicrobial action is provided by phenol-soluble modulins derived from Staphylococcus epidermidis, a normal resident of the skin", Journal of Investigative Dermatology, vol. 130, no. 1, 1 January 2010, pages 192-200, discloses the use of phenol-soluble modulins γ and δ produced by *S*. *epidermidis* as dermal antibiotics against *S*. *aureus* and *Staphylococcus.* WO2016/179440 A2 discloses a topical probiotic composition comprising one or more bacterial strains of the genus *Staphylococcus,* wherein the composition is formulated for the topical treatment of disorders of dysbiosis of the skin, scalp or mucosae.
Bhatia et al. "Propionibacterium acnes and chronic diseases", in "The infectious etiology of chronic diseases: defining the relationship, enhancing the research and mitigating the effects", 1 January 2004, Washington (DC), pages 74-80, discloses that *P. acnes* (also *C*. *acnes*) causes acne in humans.
Li et al. "Phenol-soluble modulins: novel virulence associated peptides of Staphylococci", Future Microbiology, vol. 9, no. 2, 3 December 2013, pages 203-216, discloses the use of phenol-soluble modulins from *Staphylococcus* species as dermal antibiotics. WO2015/097061 A1 discloses a method for identifying MRSA by classifying bacteria in a sample as *Staphylococcus aureus* and determining the presence or absence of phenol-soluble modulin as an indicator of the presence of the bacteria.
Rohit et al. "Genome analysis of Staphylococcus capitis TE8 reveals repertoire of antimicrobial peptides and adaptation strategies for growth on human skin", Scientific Reports, vol. 7, no. 1, 5 September 2017, pages 1-10, discloses the antibacterial effects of *Staphylococcus capitis* TE8 strain against *Bacillus subtilis, Micrococcus luteus* and *Staphylococcus aureus.* Specifically, the antibacterial activity of 1-butanol extract of S. *capitis* TE8 was tested against the 3 bacterial strains, as well as the antibacterial activity of synthetic versions of PSMβ peptides (PSMβ1-β6) and peptide HTP2388 against *M. luteus.*

### SUMMARY

The present invention is defined by the following: In a first aspect of the invention, there is provided a topical probiotic composition comprising one or more bacterial strains for use in treating a dermatological disease or disorder associated with *Cutibacterium acnes* (*C. acnes*), wherein the one or more bacterial strains comprises *Staphylococcus capitis* (*S. capitis*) N030_E12, *Staphylococcus epidermidis* (*S. epidermidis*) N009_G7, S. *epidermidis* N018_F3, or any combination thereof, and wherein at least one of the one or more bacterial strains is capable of expressing a peptide comprising a sequence selected from the group consisting of:
MTKLAEAIANTVKAGQDHDWAKLGTSIVGIAENGIGLLGKVFGF (SEQ ID NO: 9437),
MTKLAEAIANTVKAGQDHDWAKLGTSIVGIAENGIGALSKIFGG (SEQ ID NO: 9439),
MQKLAEAIANTVKAGQDHDWAKLGTSIVGIAENGINAITKIFGG (SEQ ID NO: 9441),
MTKLAEAIANAVKAGQDQDWAKLGTSIVGIAENGISLLGKVFGF (SEQ ID NO: 9443),
MQKLAEAIANTVKAGQDHDWTKLGTSIVDIVENGVSALTKVFGG (SEQ ID NO: 9445), and
MEKLFDAIRNTVDAGINQDWTKLGTSIVDIVDNGVKVISKFIGA (SEQ ID NO: 9447).

It is envisaged that the topical probiotic composition for use is formulated for the topical treatment of disorders of dysbiosis of the skin, scalp, or mucosae; wherein the topical probiotic composition inhibits the growth of *C*. *acnes;* and wherein the one or more bacterial strains is provided in a live form.

It is also envisaged that the topical probiotic composition for use is formulated for the topical treatment of disorders of dysbiosis of the skin, scalp, or mucosae; wherein the topical probiotic composition inhibits the growth of *C*. *acnes;* and wherein the one or more bacterial strains is provided in a lyophilized or freeze-dried or spray-dried form.

In an embodiment, in the topical probiotic composition for use, one or more bacterial strains can be reconstituted into a live form.

In an embodiment, the topical probiotic composition for use comprises a thickened topical formulation of the one or more bacterial strains; wherein the topical probiotic composition is formulated for the topical treatment of disorders of dysbiosis of the skin, scalp, or mucosae; wherein the topical probiotic composition inhibits the growth of *C*. *acnes;* and optionally, wherein the composition comprises a prebiotic compound, a protectant, humectant, emollient, abrasive, salt, and/or surfactant.

It is envisaged that the topical probiotic composition for use, is formulated for the treatment of disorders of dysbiosis of the skin, scalp, or mucosae, and/or wherein the composition is formulated as a cream, ointment, unguent, spray, powder, oil, thickened formulation, or poultice.

It is also envisaged that the topical probiotic composition for use inhibits C. acnes growth, viability, or activity.

In an embodiment, the topical probiotic composition is for use in treating a dermatological disease or disorder selected from acne, chronic blepharitis, and/or endophthalmitis.

In a second aspect of the invention, there is provided a pharmaceutical composition comprising a plurality of phenol-soluble modulin peptides (PSM), comprising PSMβ1 (SEQ ID NO: 9437), PSMβ3 (SEQ ID NO: 9441), PSMβ4 (SEQ ID NO: 9443), and PSMβ6 (SEQ ID NO: 9447); optionally, the plurality of phenol-soluble modulin peptides (PSM) comprises PSMβ1 (SEQ ID NO: 9437), PSMβ2 (SEQ ID NO: 9439), PSMβ3 (SEQ ID NO: 9441), PSMβ4 (SEQ ID NO: 9443), PSMβ5 (SEQ ID NO: 9445) and PSMβ6 (SEQ ID NO: 9447); and further optionally, a pharmaceutically acceptable carrier.

It is envisaged that the pharmaceutical composition comprises one or more bacteria selected from the group consisting of *S*. *capitis* N030_E12, *S. epidermidis* N009_G7, and *S*. *epidermidis* N018_F3.

It is envisaged that the pharmaceutical composition comprises a cell-free fermentation extract obtained from a culture of *S*. *capitis* N030_E12, *S. epidermidis* N009_G7, *S. epidermidis* N018_F3, or any combination thereof.

It is further envisaged that the pharmaceutical composition is for use in treating a dermatological disorder associated with *C*. *acnes,* skin or mucosal infections, atopic dermatitis, psoriasis, mastitis, acne, chronic blepharitis and endophthalmitis, or other disorders related to skin dysbiosis in humans or other mammals, wherein the composition is for application to the skin or mucosa in an effective amount of the pharmaceutical composition to a subject in need thereof.

In an embodiment, the pharmaceutical composition for use is for application topically, and wherein the pharmaceutical composition is formulated as a cream, ointment, unguent, spray, powder, oil, a lotion, tincture, thickened formulation, or poultice.

In a further embodiment, the pharmaceutical composition for use inhibits *C*. *acnes* growth, viability, or activity.

The details of one or more embodiments of the invention are set forth in the accompanying drawings and the description below. Other features, objects, and advantages of the invention will be apparent from the description and drawings, and from the claims.

### DESCRIPTION OF DRAWINGS

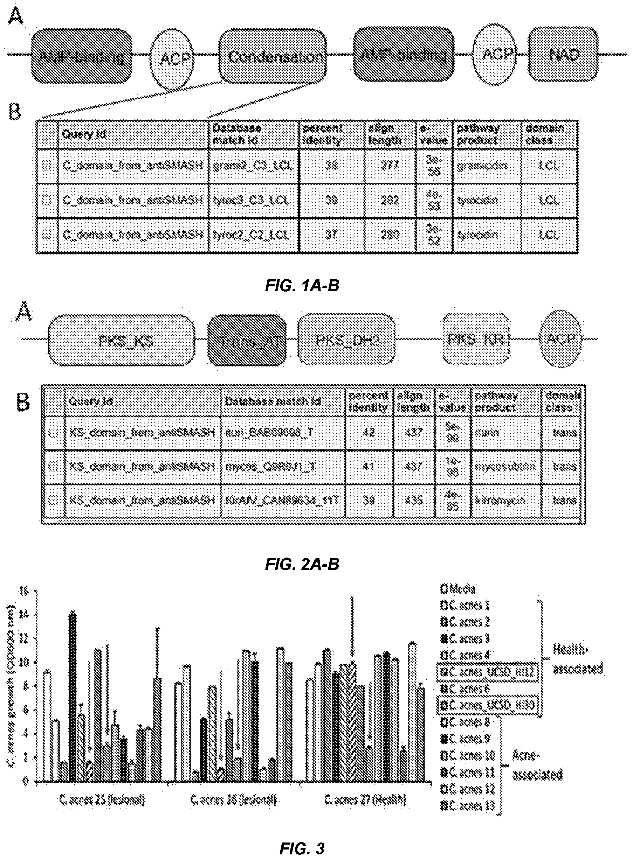

**Figure 1A-B** shows domain architecture of predicted NRPS cluster in *S*. *capitis* N030_E12. **A.** Several genes contain domains involved in synthesis of antimicrobial peptides. AMP-binding domains suggest acyl CoA synthetase activity upstream of an acyl carrier protein (ACP). The condensation domain is found in many multi-domain enzymes that synthesize peptide antibiotics, catalyzing a condensation reaction to form peptide bonds in non-ribosomal peptide biosynthesis. Nicotinamide adenine dinucleotide (NAD)- binding domain is also present which contains the short chain dehydrogenases/reductases (SDR) superfamily domain. **B.** Results of sequence comparison of the *S*. *capitis* gene containing a condensation domain against known reference genes using Natural Product Domain Seeker (NaPDos) - a bioinformatic tool for the rapid detection and analysis of secondary metabolite genes in bacteria, revealing similarity with known antimicrobial products.
**Figure 2A-B** shows domain architecture of predicted *trans*-AT PKS cluster in *S*. *epidermidis* strains. **A.** Polyketide synthases (PKS) polymerize simple fatty acids into a large variety of natural products, called polyketides. Notable domains within the PKS enzymes include dehydratase (DH) and ketoreductase (KR). **B.** Results of sequence comparison of the *S*. *epidermidis* PKS_KS-containing gene against known reference genes using NaPDos revealing similarity with known antimicrobial products.
**Figure 3** shows growth inhibition of *C*. *acnes* in sterile supernatant of multiple different *C*. *acnes* strains. Growth of lesional acne-associated strains (*C. acnes* 25 and 26) and a health-associated strain (*C. acnes* 27) was measured by OD₆₀₀ after 72 hours growth in 100% sterile supernatant of indicated *C*. *acnes* strains grown for 7 days in RCM medium. Supernatant of health-associated strains *C*. *acnes*_UCSD_HI12 and *C*. *acnes* _UCSD_HI30 (single locus sequence type D1 and A5, respectively) were potent inhibitors of *C*. *acnes* growth and chosen as attractive candidates for acne biotherapy (indicated by arrows and boxes).

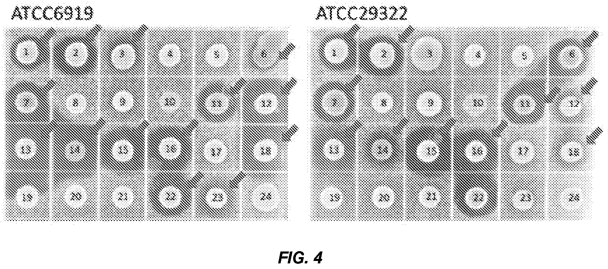

**Figure 4** shows antimicrobial activity of 24 strains of coagulase negative staphylococcus isolated from the normal human skin against 2 strains of *Proprionibacterium acnes* (ATCC6919 and ATCC29322). Anti-*P*. *acnes* activity of each CoNS clone was measured by Radial Diffusion Assay. Briefly, 5 µl of overnight culture of each CoNS clone (white spot in each square) was spotted on a Bullucella Broth agar plate containing indicated strain of *P*. *acnes.* The agar plate was incubated under an anaerobic condition at 37 °C for 72 hours to let *P. acnes* grow. The black area represents zone of growth inhibition of *P. acnes* (see arrows). Each number represents key# of CoNS strains listed in Table A.

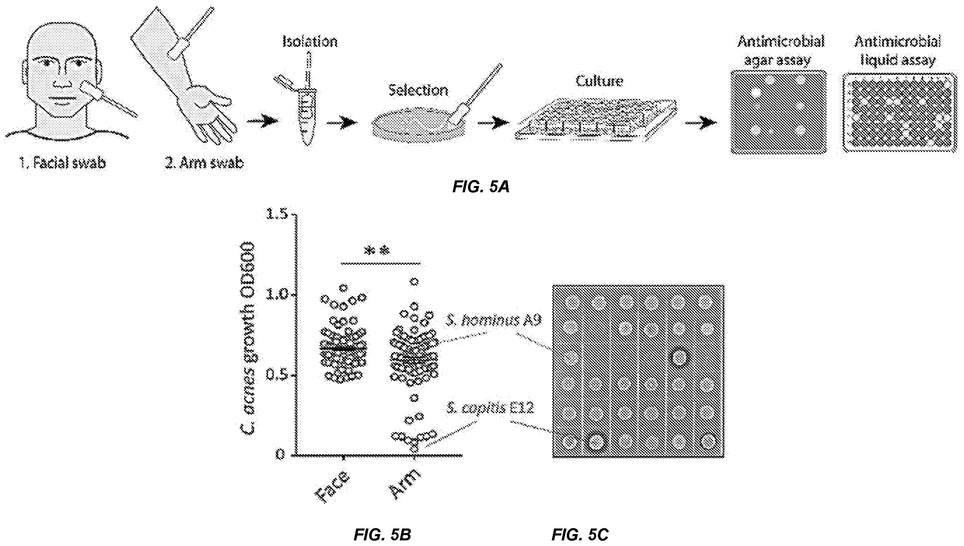

**Figure 5A-F** shows function screening method and results to identify antimicrobial CoNS species that target *C*. *acnes.* **(A)** Schematic for the high-throughput antimicrobial functional screen, outlining the collection and selection of CoNS strains from two distinct healthy skin sites and assays to detect antimicrobial activity against *C*. *acnes* via coculture on agar or growth in sterile conditioned supernatant of CoNS. **(B,C)** Growth of *C*. *acnes* after 24 h incubation in 50% sterile filtered supernatant of the CoNS strain library (B) or growth of *C*. *acnes* with CoNS coculture in agar (C). The most potent antimicrobial CoNS isolate was selected and identified as *S*. *capitis* E12 (red), whilst *S*. *hominus* A9 (blue) did not exhibit activity against *C*. *acnes.* **(D)** Table showing the inhibitory activity of *S*. *capitis* E12 and *S*. *hominus* A9, against several skin commensal and pathogen strains, including several strains of *C*. *acnes* isolated from healthy and acne skin, as measured by size of zone inhibition by antimicrobial agar assay (+ small, ++ medium, +++ large). **(E)** Growth of *C*. *acnes* C2 after 24 h incubation in media alone or increasing concentrations of sterile supernatant of *S*. *capitis* E12 or *S*. *hominus* A9. **(F)** Survival of several species of staphylococci and *C*. *acnes* after 24 h post-treatment with increasing concentrations of sterile *S*. *capitis* E12 supernatant, as measured by the number of surviving CFU plated onto the appropriate selective agar.

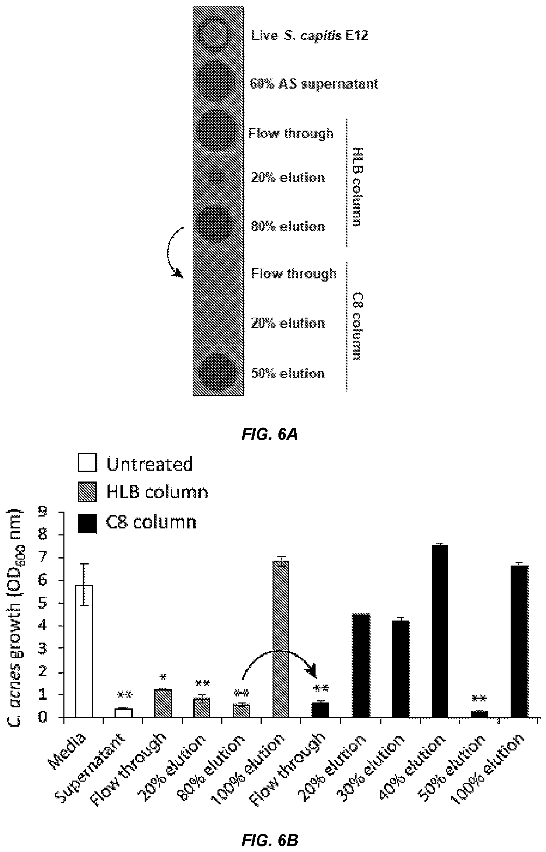

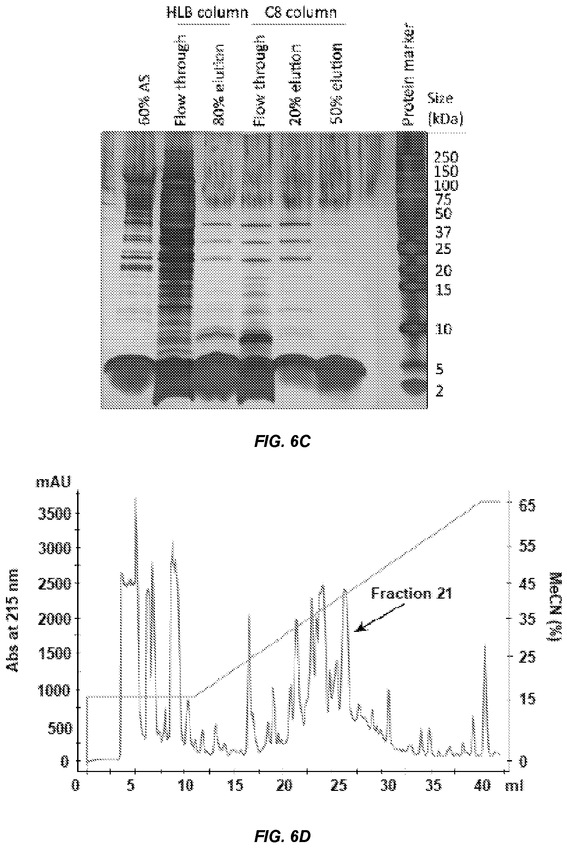

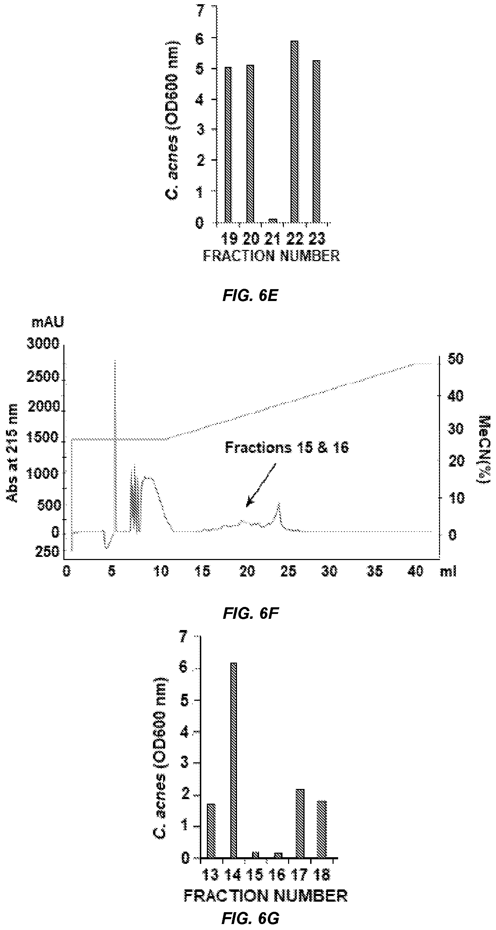

**Figure 6A-H** shows purification and identification of S. capitis E12 antimicrobial peptides. **(A,B)** 60% ammonium sulphate precipitated supernatant of *S*. *capitis* E12 was loaded onto a HLB column and eluted by 80% acetonitrile. The HLB eluant was loaded onto a C8 cartridge and eluted by 50% acetonitrile. Fractions were measured for activity against *C*. *acnes* by agar assay **(A)** and liquid culture **(B). (C)** Silver stain of the total protein content of *S*. *capitis* treated or untreated supernatant and the SPE flow-through and eluted fractions. **(D)** HPLC purification of *S*. *capitis* supernatant identified several peptide peaks, of which a single fraction (21) was identified as having anti-*C*. *acnes* activity **(E).** Fraction 21 was pooled from five separate HPLC runs and purified by second step HPLC resulting in two fractions (15 and 16) with anti-*C. acnes* activity **(G).** MS was performed for active fractions 15 and 16 including four non-active fractions as controls and identified four distinct PSMβ peptides as likely candidates for antimicrobial peptides. **(H)** Results of the top 8 peptide hits from MS detection of HPLC purified active fractions (15 and 16) and control non-active fractions (13,14, 17 and 18) revealing peptides corresponding to 'antibacterial protein' and later confirmed as PSMβ peptides.

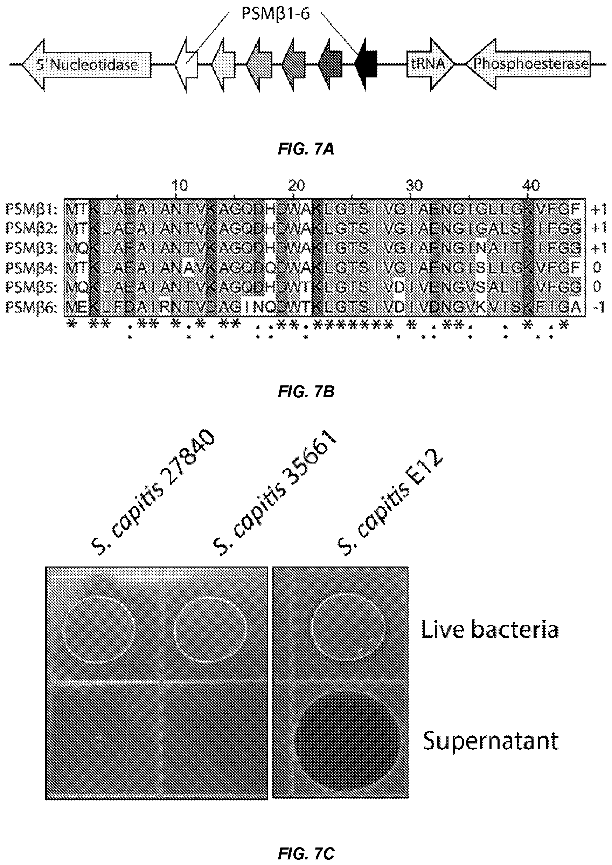

**Figure 7A-D** shows whole genome sequencing of S. capitis E12 reveals sequence and predicted properties of the PSMβ peptides. **(A)** Schematic highlighting the *S*. *capitis* E12 genetic cluster containing six gene-encoding PSMβ peptides (PSMβ1-6). **(B)** Multiple sequence alignment (ClustalW) of all six PSMβ peptides, including the predicted charge for each peptide (SEQ ID NOs:9437, 9439, 9441, 9443, 9445, 9447). **(C)** Absence of antimicrobial activity against *C. acnes* during live coculture or sterile supernatant exposure with ATCC *S*. *capitis* strains 35661 and 27840 that lack PSMβ genes. **(D)** Representative helical wheel plot (Heliquest) of PSMβ1 indicating a predicted amphipathic structure.

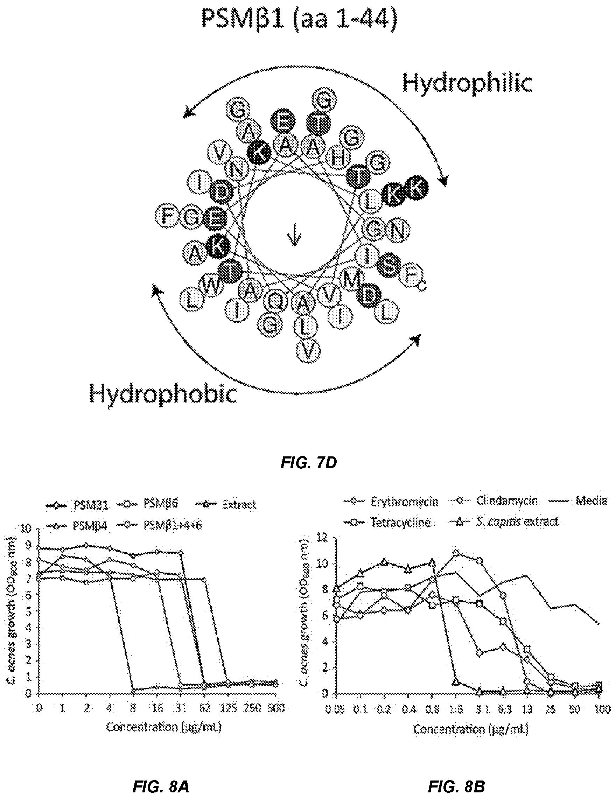

**Figure 8A-F** shows preclinical efficacy of PSMβ peptides and extract as a therapeutic for acne vulgaris. **(A)** Inhibition of *C. acnes* C2 after 72 h culture in the presence of S. *capitis* E12 extract or with synthetic peptides PSMβ1, PSMβ4, PSMβ6 individually or in combination of all three **(B)** Growth of *C*. *acnes* C2 cultured for 72 h in the presence of *S*. *capitis* extract or with several different antibiotics. **(C)** Cytotoxicity of NHEKs treated with S. *capitis* E12, *S. epidermidis* C5, *S. epidermidis* 1457 and *S*. *capitis* 35561 supernatant for 24 h, presented as the percentage of maximal LDH release. **(D)** Visual representation of erythema on the back skin of SKH1 mouse after inoculation with 1x10⁷ CFU of *S*. *capitis* E12, *S. epidermidis* C5, *S. epidermidis* 1457 and *S*. *capitis* 35661 for 24 h. **(E)** Total number of surviving CFU of *C*. *acnes* C2 on *ex vivo* pig skin explants 24 h post-treatment with *S*. *capitis* E12 extract (10 mg/mL) or PBS control. **(F)** Total number of surviving CFU of *C*. *acnes* C2 on SKH1 mouse back skin 24 h post-treatment with *S*. *capitis* E12 extract (10 mg/mL) or PBS control.

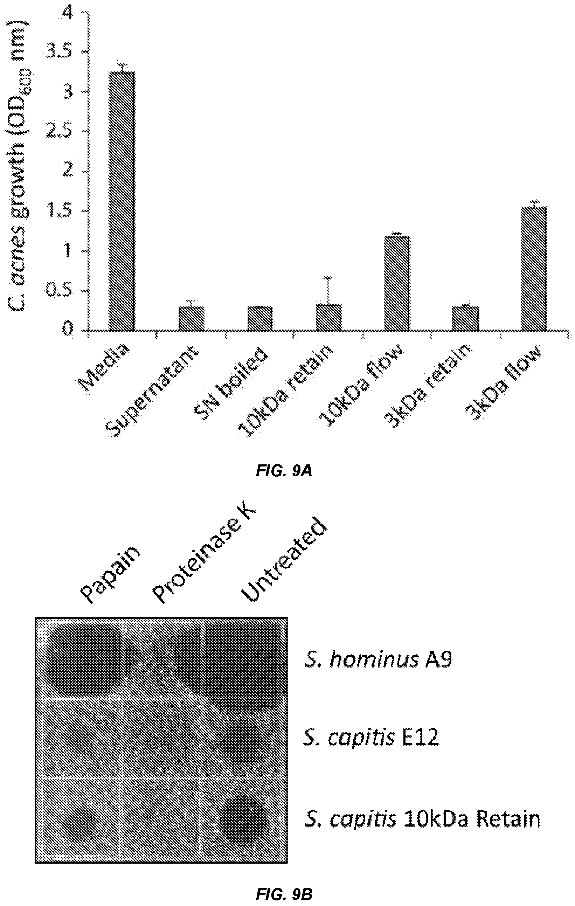

**Figure 9A-C** shows characterization of the biological nature of the *S*. capitis active molecule(s). **(A)** Growth of *C*. *acnes* C2 was measured in liquid culture with or without *S*. *capitis* E12 supernatant untreated, boiled at 90°C for 15 mins or separated through 10 kDa or 3 kDa MWCO columns into retained or flow-through fractions. **(B)** *S. capitis* E12 and control *S*. *hominus* A9 supernatant was subjected to papain or proteinase K proteolytic digestion (200 µg/ml) for 45 min at 37°C and inoculated onto *C*. acnes-containing agar to measure antimicrobial activity. **(C)** CoNS supernatant was subjected to precipitation in different saturation amounts of ammonium sulphate, and antimicrobial activity of each precipitate was measured by growth of *C*. *acnes* C2 72 h post-treatment.

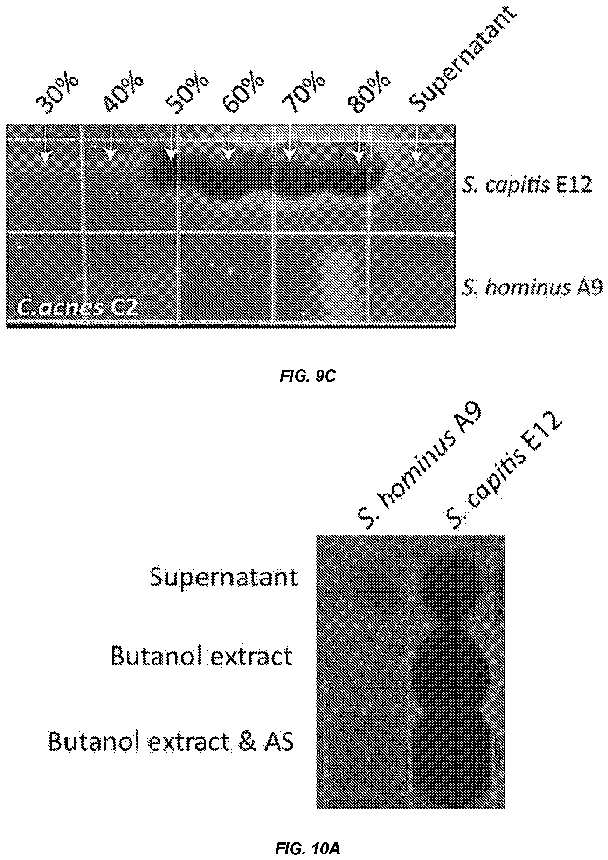

**Figure 10A-B** shows PSMβ peptides are extracted by n-Butanol treatment of S. capitis E12 supernatant. **(A)** Antimicrobial activity against *C*. *acnes* C2 is retained and enhanced after butanol extraction of PSMβ from sterile supernatant of *S*. *capitis* E12 and not with the lantibiotic-producing *S*. *hominus* A9. **(B)** Silver stain of the total protein content of *S*. *capitis* E12 supernatant before and after butanol extraction showing enrichment of small PSMβ peptides.

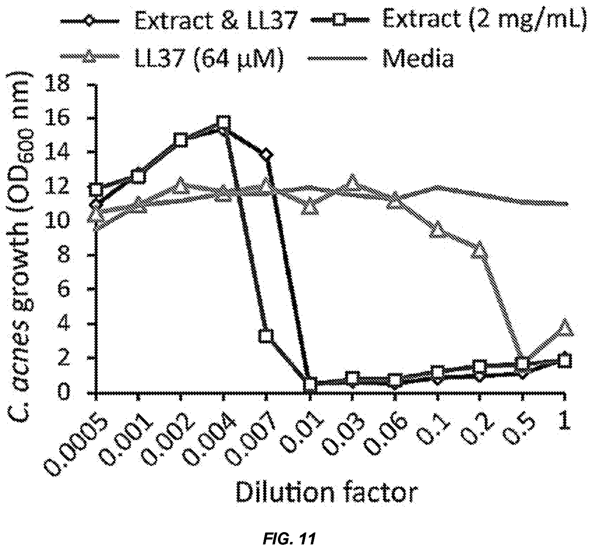

**Figure 11** shows PSMβ does not synergize with host LL-37 antimicrobial peptide. Assessment of synergistic antimicrobial activity against *C*. *acnes* C2 from of *S*. *capitis* E12 extract and human LL-37 antimicrobial peptide, at indicated concentrations.

### DETAILED DESCRIPTION

As used herein and in the appended claims, the singular forms "a," "an," and "the" include plural referents unless the context clearly dictates otherwise. Thus, for example, reference to "an agent" includes a plurality of such agents and reference to "the microorganism" includes reference to one or more microorganisms and equivalents thereof known to those skilled in the art, and so forth.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Any methods and reagents similar or equivalent to those described herein can be used in the practice of the disclosed methods and compositions.

*Cutibacterium acnes* (*C. acnes;* formerly *Propionibacterium acnes; C. acnes* and *P. acnes* are used interchangeably herein) is a slow-growing, aerotolerant anaerobic, Gram-positive bacterium linked to skin acne; it can also cause chronic blepharitis and endophthalmitis, the latter particularly following intraocular surgery. Although *P. acnes* is a member of the normal skin commensal bacterial flora, it plays a critical role in the development of inflammatory acne when the microorganism overgrows within the pilosebaceous unit. Inflammatory acne is the most common disease of human skin afflicting up to 80% of individuals through their lives.

As mentioned *C*. *acnes* plays a critical role in the development of inflammatory acne when the microorganism overgrows within the pilosebaceous unit. Inflammatory acne is the most common disease of human skin afflicting up to 80% of individuals through their lives. Acne has many different symptoms including comedones, papules, pustules, nodules, cysts and pilosebaceous inflammation. Among these, inflammatory lesions of acne are of serious concern to patients because they may lead to acne scarring, thereby inducing adverse psychological effects. The genome of the bacterium has been sequenced and a study has shown several genes can generate enzymes for degrading skin and proteins that may be immunogenic (activating the immune system). This bacterium is largely commensal and part of the skin flora present on most healthy adult human skin. Typically, the organism is just barely detectable on the skin of healthy preadolescents. It lives primarily on, among other things, fatty acids in sebum secreted by sebaceous glands in the follicles. It may also be found throughout the gastrointestinal tract.

Reduction in *P. acnes* survival correlates with clinical improvement of acne in patients. Systemic antibiotics have been used to treat acne for several decades and are still widely prescribed for acne patients. Topical antibiotics are also helpful, and the oxidizing agent benzoyl peroxide (BPO) has been one of the most frequently used topical medications for acne treatment. Topical drug therapies are often used as the first line treatment for patients suffering from mild to moderate acne. However, current antibiotic treatments have major drawbacks. Systemic antibiotics non-specifically disrupts microbial ecosystem and promote antibiotic resistance. Topical antibiotics are very poor at killing *P. acnes* on the skin surface.

The disclosure demonstrates that coagulase-negative Staphylococci (CoNS) species that normally reside on skin such as *S. epidermidis* and *S*. *capitis* protect against *C*. *acnes* by producing factors that inhibit *C*. *acnes.* The disclosure thus provides prebiotics, probiotics and postbiotics that can be used to treat *C*. *acnes* infection and/or inhibit *C*. *acnes* growth.

The disclosure shows, for example, that *S*. *capitis* E12 constitutively produces antimicrobial peptides in sufficient quantities to kill *C*. *acnes.* Strikingly, incubation of a 0.6X concentration of *S*. *capitis* E12 supernatant with a strain of *C*. *acnes* that is associated with acne resulted in greater than 4 log reduction in survival of the disease-associated bacterium, and complete sterilization of the culture after 24 h. Another attractive feature was that *C*. *acnes* C2 did not show acquisition of resistance up to 20 generations. The potency and poor capacity to induce resistance from the agents including, for example, PSMs, produced by *S*. *capitis* E12 is consistent with the mode of action of the αhelix, amphipathic structure of these peptides. Such a structure is common among many antimicrobial peptides, and enables insertion into and destabilization of bacterial membranes. Such a mechanism of direct membrane disruption is ideal for an antimicrobial that works on an epithelial surface.

While the *S*. *capitis* genome contains a cluster of up to six PSM-encoding genes, PSMβ1, 3, 4 and 6 were readily detected by MS analysis of the purified active fractions. Antimicrobial assays with synthetic peptides PSMβ1, 4 and 6 revealed greater activity against *C*. *acnes* when the bacteria were treated with a combination of all three. Likewise, the expected overall charge of each PSMβ is subtly different, thus likely impacting binding capacity to a hydrophobic column as well as interaction with the bacterial membrane. Individually, the synthetic PSM peptides were far less potent than the extract. It is contemplated that PSMβ2 and PSMβ5 may also be secreted by *S*. *capitis* but eluted in different fractions that were not biologically active by themselves but would increase the activity of PSMβ1, 3, 4 and 6. The β class PSMs have been found in other staphylococci and typically number between two and four. However, their exact role has remained elusive and unlike the smaller PSMα, are not thought to be involved in virulence. *S*. *aureus* contains two βPSMs (PSMβ1/2), but neither are reported to be antimicrobial. Other studies have shown other synthetic PSMβ peptides to be inhibitory against *S*. *aureus in vitro* but not known to be active against *C*. *acnes.* Therefore, adopting the present screening approach identified activity that would not have been detectable by using a genetic screening approach alone. The optimum killing efficacy and specificity of the combination, rather than individual peptides, suggests that a therapeutic approach combining multiple PSMs or the live organism itself may be most effective. The disclosure thus shows that the skin microbiome is a valuable resource for discovering new antimicrobials that can be remarkably selective against specific pathogens and valuable in combating skin disease such as acne vulgaris.

The term "contacting" refers to exposing the skin to a topical prebiotic, probiotic and/or postbiotic composition such that the composition can kill or inhibit *C*. *acnes* on the skin.

As used herein, the term "fermentation extract" means a product of fermenting a probiotic commensal skin bacteria in a culture and under appropriate fermentation conditions. For example, culturing *S*. *capitis* N030_E12, *S. epidermidis* AMT5_C5, *S*. *epidermidis* N009_G7 and/or *S*. *epidermidis* N018_F3 can produce factors useful for inhibiting *C. acnes* growth and survival. An extract from *S*. *capitis* N030_E12, *S. epidermidis* AMT5_C5, *S*. *epidermidis* N009_G7 and/or *S*. *epidermidis* N018_F3 can be applied to the skin to inhibit *C*. *acnes* growth and/or infection, and/or treat acnes vulgaris. In some embodiments, the fermentation extract comprises one or more of PSM selected from the group consisting of PSMβ-1, -2, -3, -4, -5, -6 and any combination thereof.

The terms "inhibiting" or "inhibiting effective amount" refers to the amount of prebiotic, probiotic and/or postbiotic skin composition comprising, consisting essentially of or consisting of one or more probiotic microorganism and/or fermented medium or extract and/or fermentation by-products (*e.g.*, PSMβs) and/or synthetic molecules that is sufficient to cause, for example, inhibition of *C*. *acnes* growth, proliferation or presence on the skin. The term "inhibiting" also includes preventing or ameliorating a sign or symptoms of a disorder (*e.g.,* acne, sore, and the like).

The term "phenol soluble modulin" or "PSM" refers to a family of protein toxins that are soluble in phenols and that are produced by staphylococcus bacteria. The protein sequences of the PSMs vary. In the present invention a set of PSMs are identified that inhibit the growth and viability of C. acnes. These PSMs are identified in Fig. 7B (*e.g.,* PSMβ1-6). PSMs of the invention can be synthesized using a peptide synthesizer or purified from fermentation extracts of cultures using for example, *S. capitis* E12. Alternatively, the invention provides the coding sequences for the PSMs of the invention. Such coding sequences can be used to generate PSMs by recombinant molecular biology techniques. For example, one of skill in the art can insert the coding sequence of one or more PSMs into an expression vector and then transfect or transform a suitable host cell (*e.g.,* a bacterial cell), with the vector such that that recombinant bacteria cell expressed the one or more PSMs. Such recombinant bacteria cells can be used in a probiotic of the invention or can be used to produce a postbiotic (*e.g.,* an extract) suitable for the methods described herein in the treatment of acne.

As used herein, the term "postbiotic", "postbiotic composition" or "topical postbiotic composition" or "postbiotic skin composition" refer to the non-viable bacterial products or metabolic byproducts from the probiotic organism comprising a probiotic commensal skin bacteria fermentation extract and a pharmaceutical carrier. In certain embodiments, the postbiotic comprises at least 2, at least 3, at least 4 of PSMβ1, 3, 4, and/or 6.

As used herein, "polynucleotide" refers to a polymer of deoxyribonucleotides or ribonucleotides, in the form of a separate fragment or as a component of a larger genetic construct (*e.g.*, by operably linking a promoter to a polynucleotide encoding a peptide of the invention). Numerous genetic constructs (*e.g.*, plasmids and other expression vectors) are known in the art and can be used to produce the peptides of the invention in cell-free systems or prokaryotic or eukaryotic (*e.g.*, yeast, insect, or mammalian) cells. By taking into account the degeneracy of the genetic code, one of ordinary skill in the art can readily synthesize polynucleotides encoding the peptides of the invention. The polynucleotides of the invention (those set forth in the accompanying sequence listing) can readily be used in conventional molecular biology including for the generation of probes, primers and expression constructs.

As used herein a "prebiotic" is a compound or agent that stimulate the growth and or activity of an *S*. *epidermidis* and/or *S*. *capitis* organism of the invention. For example, a subject afflicted with acne or a *C*. *acnes* infection can apply a prebiotic to, *e.g.,* their skin such that the prebiotic stimulates the growth and activity of an *S*. *epidermidis* and/or *S*. *capitis* of the invention. The stimulated growth and activity of the *S*. *epidermidis* and/or *S*. *capitis* would thus produce an inhibitory effect on the *C*. *acnes* infection thereby treating the acne. A prebiotic compound can comprise a polysaccharide, hydrolysate, salt, herbal extract, or any other compound sufficient to foster the growth of an associated probiotic strain (*e.g., S. capitis* N030_E12, *S. epidermidis* AMT5_C5, *S. epidermidis* N009_G7, *S*. *lugdnensis* N028_E7, and/or *S*. *epidermidis* N018_F3) when used in combination with that strain, such as yeast hydrolysate in concentrations of less than about 40% (w/w), microcrystalline cellulose in concentrations of less than about 10% (w/w), and/or sucrose in concentrations of less than about 10% (w/w). Other examples of prebiotics that may be adapted for use with cutaneous bacteria include inulin, glucooligosaccharides, isomaltooligosaccharides, lactosucrose, polydextrose, soybean oligosaccharides, and xylooligosaccharides, and those disclosed in Gibson, G.R. and Roberfroid, M, (Eds.) Handbook of Prebiotics, CRC press (2008); Roberfroid, M., J. Nutr. 137(3):830S-837 (2007) and Slavin, J. Nutrients 5(4):1417-1435 (2013).

As used herein, the term "probiotic", "probiotic composition" or "topical probiotic composition" or "probiotic skin composition" comprises a probiotic commensal skin bacteria, an attenuated or engineered microorganism that expresses an agent (*e.g.,* at least two or more of the PSMβ described herein) that inhibits *C*. *acnes* growth or infection, and a pharmaceutical carrier that maintain the viability of the commensal skin bacteria. The probiotic of the disclosure may comprise a suitable pharmaceutically acceptable carrier for formulation component and contain a probiotic commensal bacteria of the disclosure at a purification of at least 50-80%, 85%, 87%, 90%, 92%, 95%, 98% or 100%.

In some embodiments, a probiotic composition described herein comprises a probiotic organism. In further embodiments, the probiotic organism is a bacterium. In further embodiments, the bacterium comprises a component of the normal skin flora. The bacterium of the disclosure may comprise a strain of *Staphylococcus hominis.* In other embodiments, the bacterium comprises a strain of *Staphylococcus epidermidis.* In yet another embodiment, the bacterium comprises *Staphylococcus capitis.* The bacterium of the disclosure may comprise *Staphylococcus lugdnensis.* In other embodiments, the probiotic comprises a mixture of strains. The mixture of strains of the disclosure may comprise multiple strains of *S*. *hominis.* In other embodiments, the mixture of strains comprises multiple strains of *S*. *epidermidis.* In other embodiments, the mixture of strains comprises multiple strains of *S. capitis.* The mixture of strains of the disclosure may comprise multiple strains of *S*. *lugdnensis* and/or one or more strains of *S*. *capitis* and/or one or more strains of *S*. *lugdnensis.* The mixture of strains of the disclosure may comprise one or more strains of S. *hominis* and one or more strains of *S*. *epidermidis.* The composition of the disclosure may comprise one or more strains in addition to *S. hominis, S. epidermidis, S. capitis* and/or *S*. *lugdnensis.* The additional strain or strains of the disclosure may comprise one or more strains from the genus *Staphylococcus, Lactobacillus* or *Lactococcus.* Specific probiotic formulations may comprise *S*. *capitis* N030_E12, *S. epidermidis* AMT5_C5, *S. epidermidis* N009_G7, *S. lugdnensis* N028_E7, and/or *S*. *epidermidis* N018_F3. Such formulations typically comprise sufficient quantities of bacterial cells as to provide a final density of 10³⁻10⁶ CFU/cm² when applied to the skin of a subject. Such formulations may comprise concentrations of from about 10⁴ to about 10⁷ CFU/g, or alternatively, from 10 to about 10⁵ CFU/g, or alternatively, from about 10⁵ to about 10⁹ CFU/g. Such formulations of the disclosure may comprise multiple strains of S. *hominis, S. epidermidis, S. capitis,* and/or *S*. *lugdnensis,* and may further comprise *Lactococcus lactis, Lactobacillus plantarum, Lactobacillus rhamnosus, Lactobacillus acidophilus,* and/or other such species or strains as are known in the art to form a part of the normal healthy cutaneous or mucosal flora. In the disclosure, *S. hominis* (*e.g.,* strains - A12, -C2, -D12, and/or -G1 as set forth in Table A) may comprise 100% of the bacterial cells in a formulation. Further, an *S*. *hominis* strain may comprise 90-100%, 85-95%, 70-80%, 75-85%, 60-70%, 65-75%, 50-60%, 55-65%, 40-50%, 45-55%, 30-40%, 35-45%, 20-30%, 25-35%, 10-20%, 15-20%, 1-10%, 5-15%, or less than 1% of the bacterial cells in a given formulation, wherein the remainder of the colony forming units are provided by *S*. *epidermidis, S. capitis,* and/or *S*. *lugdnensis,* or *Lactococcus lactis, Lactobacillus plantarum, Lactobacillus rhamnosus, Lactobacillus acidophilus,* and/or other such strains as are known in the art to form a part of the normal healthy cutaneous or mucosal flora. In some embodiments, *S. epidermidis* strains (*e.g.*, -N018-F3, -G6, -C5 and/or -N038F6 as set forth in Table A) comprise 100% of the bacterial cells in a formulation. In some further embodiments, *S. epidermidis* comprises 90-100%, 85-95%, 70-80%, 75-85%, 60-70%, 65-75%, 50-60%, 55-65%, 40-50%, 45-55%, 30-40%, 35-45%, 20-30%, 25-35%, 10-20%, 15-20%, 1-10%, 5-15%, or less than 1% of the bacterial cells in a given formulation, wherein the remainder of the colony forming units are provided by *S*. *hominis, S. capitis,* and/or *S*. *lugdnensis,* or *Lactococcus lactis, Lactobacillus plantarum, Lactobacillus rhamnosus, Lactobacillus acidophilus,* and/or other such strains as are known in the art to form a part of the normal healthy cutaneous or mucosal flora. In the disclosure, *S*. *capitis* strain N030-H8 may comprise 100% of the bacterial cells in a formulation. In some further embodiments, *S*. *capitis* comprises 90-100%, 85-95%, 70-80%, 75-85%, 60-70%, 65-75%, 50-60%, 55-65%, 40-50%, 45-55%, 30-40%, 35-45%, 20-30%, 25-35%, 10-20%, 15-20%, 1-10%, 5-15%, or less than 1% of the bacterial cells in a given formulation, wherein the remainder of the colony forming units are provided by *S*. *hominis, S. epidermidis,* and/or *S*. *lugdnensis,* or *Lactococcus lactis, Lactobacillus plantarum, Lactobacillus rhamnosus, Lactobacillus acidophilus,* and/or other such strains as are known in the art to form a part of the normal healthy cutaneous or mucosal flora.

In the disclosure, bacteria other than *S*. *hominis, S. epidermidis, S. capitis* and/or *S*. *lugdnensis* may comprise about 50% or less of the bacterial cells in the formulation. In the disclosure said bacteria may comprise less than 50%, less than 40%, less than 30%, less than 20%, less than 10%, less than 5%, or less than 1% of the bacterial cells within a given formulation. In the disclosure, bacteria other than *S*. *hominis, S. epidermidis, S. capitis* and/or *S*. *lugdnensis* may comprise *Lactococcus lactis, Lactobacillus plantarum, Lactobacillus rhamnosus, Lactobacillus acidophilus,* and/or other such species or strains as are known in the art to form a part of the normal healthy cutaneous or mucosal flora. In some embodiments, the formulations can comprise a combination of 2 or 4 of the strains *S*. *hominis, S. epidermidis, S. capitis* and/or *S*. *lugdnensis* in any percentage from about 1%-99% of a particular strain (*e.g.*, about 25% *S*. *hominis,* 25% *S*. *epidermidis,* 25% *S*. *capitis* and 25% *S*. *lugdnensis*)*.* Any percentage or ratio of any 2 or more (*e.g.,* 2, 3, 4) of the strains showing positive inhibition of C. acnes are contemplated herein. In some embodiments, the formulations comprise about 50% *S*. *capitis* of the strains listed above and about 50% *S*. *epidermidis* of the strains listed above. In some embodiments, the formulations comprise about 40% *S*. *capitis* of the strains listed above and about 60% *S*. *epidermidis* of the strains listed above. In some embodiments, the formulations comprise about 70% *S*. *capitis* of the strains listed above and about 30% *S*. *epidermidis.* In some embodiments, the formulations comprise about 30% *S*. *capitis* of the strains listed above and about 70% *S*. *epidermidis* of the strains listed above. In some embodiments, the formulations comprise about 80% *S*. *capitis* of the strains listed above and about 20% *S*. *epidermidis* of the strains listed above. In some embodiments, the formulations comprise about 20% *S*. *capitis* of the strains listed above and about 80% *S*. *epidermidis* of the strains listed above. In some embodiments, the formulations comprise about 90% *S*. *capitis* of the strains listed above and about 10% *S*. *epidermidis* of the strains listed above. In some embodiments, the formulations comprise greater than about 90% *S*. *capitis* of the strains listed above and less than about 10% *S*. *epidermidis* of the strains listed above. In some embodiments, the formulations comprise less than about 10%S. *capitis* of the strains listed above and greater than about 90% S. *epidermidis* of the strains listed above.

As used herein, the term "Probiotic Commensal Skin Bacteria" includes a microorganism of the skin microbiome. The probiotic commensal skin bacteria can include a composition of bacterial that inhibits *C*. *acnes* growth or infection or kills *C*. *acnes.* In one embodiment, a probiotic commensal skin bacteria comprises one or more bacteria selected from the group consisting of is *S*. *capitis* N030_E12, *S. epidermidis* AMT5_C5, *S*. *epidermidis* N009_G7 and S. *epidermidis* N018_F3.

The term "purified" and "substantially purified" as used herein refers to cultures, or co-cultures of microorganisms or of biological agent (*e.g.* fermentation media and extracts, fractionated fermentation media, fermentation by-products, peptide, polypeptide, gene, polynucleotide etc.) that is substantially free of other cells or components found in the natural environment with which an *in vivo*-produced agent would naturally be associated. The level of purification can vary from about 50-80% pure for a certain microorganism or agent to 100% pure of a certain microorganism or agent.

The term "therapeutically effective amount" as used herein for treatment of a subject afflicted with a disease or disorder resulting from overgrowth or infection by *C*. *acnes* means an amount of a prebiotic, probiotic and/or postbiotic skin composition or extract thereof sufficient to ameliorate a sign or symptom of the disease or disorder. For example, a therapeutically effective amount can be measured as the amount sufficient to decrease a subject's symptoms of acne. Typically, the subject is treated with an amount to reduce a symptom of a disease or disorder by at least 50%, 90% or 100%. Generally, the optimal dosage will depend upon the disorder and factors such as the weight of the subject, the type of bacteria, the sex of the subject, and degree of symptoms. Nonetheless, suitable dosages can readily be determined by one skilled in the art.

As used herein, the term "topical" can include administration to the skin externally, as well as shallow injection (*e.g.,* intradermally and intralesionally) such that a topical probiotic composition described herein comes in direct contact with skin and dermal layer.

The invention provides whole cell preparations comprising a substantially homogeneous or substantially pure preparation of *S*. *capitis* N030_E12, *S. epidermidis* AMT5_C5, *S*. *epidermidis* N009_G7 and/or *S*. *epidermidis* N018_F3. Such a preparation can be used in the preparation of compositions for the treatment of acne, inflammation and microbial infections. Whole cell preparation can comprise *S*. *capitis* N030_E12, *S. epidermidis* AMT5_C5, *S. epidermidis* N009_G7 and/or *S*. *epidermidis* N018_F3. The disclosure also provides fractions derived from such whole cells comprising agents that reduce *C*. *acnes* growth or viability in the skin.

The ability of a first bacterial composition (*e.g., S. capitis*) to inhibit the activity of a second bacterial composition (*e.g., C. acnes*) can be determined by contacting, *e.g.,* a probiotic or postbiotic with, for example, a *C. acnes* and measuring the growth or viability of the *C*. *acnes* before and after contacting the probiotic or postbiotic. Contacting of an organism with a topical probiotic composition of the disclosure can occur *in vitro,* for example, by adding the topical probiotic or postbiotic composition to a bacterial culture. Alternatively, contacting can occur *in vivo,* for example by contacting the topical probiotic or postbiotic composition with a subject afflicted with a skin disease or disorder.

A probiotic commensal skin bacterial preparation or postbiotic composition can be prepared in any number of ways. In one embodiment, a probiotic commensal skin bacterial preparation is prepared by culturing and isolating a bacterial strain selected from the group consisting of *S*. *capitis* N030_E12, *S. epidermidis* AMT5_C5, *S. epidermidis* N009_G7, *S. epidermidis* N018_F3 and any combination thereof and resuspending the bacterial strain(*s*) in a suitable carrier. In another embodiment, a postbiotic composition is prepared by culturing a commensal bacterial strain selected from the group consisting of *S*. *capitis* N030_E12, *S. epidermidis* AMT5_C5, *S. epidermidis* N009_G7, *S. epidermidis* N018_F3 and any combination thereof under condition to obtain a fermentation product, obtaining a cell-free preparation of the fermentation extract and combining the extract with a suitable carrier.

Any of a variety of methods known in the art can be used to administer a topical probiotic or postbiotic compositions to a subject. For example, a probiotic or postbiotic skin composition or extract or synthetic preparation of the invention may be formulated for topical administration (*e.g.,* as a lotion, cream, spray, gel, or ointment). Such topical formulations are useful in treating or inhibiting microbial, fungal, viral presence or infections or inflammation on the skin. Examples of formulations include topical lotions, creams, soaps, wipes, and the like.

In yet another embodiment, a topical probiotic composition is provided that comprises a plurality of probiotic commensal skin bacteria. When used for the treatment of acne or other skin diseases or disorders associated with increased C. *acne* levels or activity, the composition comprises one or more bacteria that inhibit such *C*. *acnes* on the skin. In such instances, the probiotic commensal skin bacteria is a coagulase negative *Staphylococcus* sp. In one embodiment, the probiotic commensal skin bacteria is selected from the group consisting of *S*. *capitis* N030_E12, *S. epidermidis* AMT5_C5, *S. epidermidis* N009_G7, S. *epidermidis* N018_F3 and any combination thereof.

In another embodiment, the topical probiotic composition comprises a postbiotic commensal skin bacteria fermentation extract that inhibits *C*. *acnes* growth or activity on the skin. In various aspects, the bacteria from which the extract is produced comprises *S. capitis* N030_E12, *S. epidermidis* AMT5_C5, *S. epidermidis* N009_G7 and/or S. *epidermidis* N018_F3.

In accordance with a further embodiment, the topical compositions above can be formulated as a lotion, shake lotion, cream, ointment, gel, foam, powder, solid, paste or tincture.

In the disclosure, a bandage or dressing is provided comprising the topical prebiotic, probiotic and/or postbiotic compositions described above. Further, a bandage or dressing is provided the major constituents of which includes a matrix and a probiotic commensal skin bacteria that inhibits *C*. *acnes* on the skin. Even further, a bandage or dressing is provided the major constituents of which includes a matrix and a postbiotic that inhibits *C*. *acnes* on the skin.

A pharmaceutical composition comprising a probiotic skin composition disclosed herein comprising a commensal bacteria (*e.g., S. capitis* N030_E12, *S. epidermidis* AMT5_C5, *S. epidermidis* N009_G7 and/or S. *epidermidis* N018_F3), may be formulated in any dosage form that is suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation comprising a probiotic disclosed herein may also comprise liposomes, micelles, microspheres, nanosystems, and mixtures thereof.

A "pharmaceutically acceptable carrier" is intended to include solvents, dispersion media, coatings, antibacterial and antifungal agents (as needed so long as they are not detrimental to the probiotic commensal bacteria), isotonic and absorption delaying agents, and the like. The use of such media and agents for pharmaceutically active substances is well known in the art. Except insofar as any conventional media or agent is incompatible with the pharmaceutical composition, use thereof in the therapeutic compositions and methods of treatment is contemplated. Supplementary active compounds can also be incorporated into the compositions.

Pharmaceutically acceptable carriers and excipients suitable for use in the topical formulations disclosed herein include, but are not limited to, aqueous vehicles, water-miscible vehicles, non-aqueous vehicles, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryopretectants, lyoprotectants, thickening agents, and inert gases.

A pharmaceutical composition comprising a probiotic may be formulated in the forms of ointments, creams, sprays and gels. Suitable ointment vehicles include oleaginous or hydrocarbon vehicles, including such as lard, benzoinated lard, olive oil, cottonseed oil, and other oils, white petrolatum; emulsifiable or absorption vehicles, such as hydrophilic petrolatum, hydroxystearin sulfate, glycerol and anhydrous lanolin; water-removable vehicles, such as hydrophilic ointment; water-soluble ointment vehicles, including polyethylene glycols of varying molecular weight; emulsion vehicles, either water-in-oil (W/O) emulsions or oil-in-water (O/W) emulsions, including cetyl alcohol, glyceryl monostearate, lanolin, and stearic acid (see, Remington: The Science and Practice of Pharmacy). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain material substantially uniformly throughout the liquid carrier. Suitable gelling agents include crosslinked acrylic acid polymers, such as carbomers, carboxypolyalkylenes, Carbopol^{RTM}; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

A pharmaceutical composition comprising a prebiotic, probiotic and/or postbiotic disclosed herein can be formulated either alone or in combination with one or more additional therapeutic agents, including, but not limited to, chemotherapeutics, antibiotics (so long as they do not destroy the probiotic benefits), antifungal-agents, anti-pruritics, analgesics, protease inhibitors and/or antiviral agents.

Topical administration, as used herein, include (intra)dermal, conjunctival, intracorneal, intraocular, ophthalmic, auricular, transdermal, nasal, vaginal, urethral, respiratory, and rectal administration. Such topical formulations are useful in treating or inhibiting infections of the eye, skin, and mucous membranes (*e.g.,* mouth, vagina, rectum). Examples of formulations in the market place include topical lotions, creams, soaps, wipes, and the like.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient disclosed herein, a propellant as solvent; and/or a surfactant, such as sorbitan trioleate, oleic acid, or an oligolactic acid.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT , Rohm America, Inc., Piscataway, N.J.); poly(2-hydroxyethyl-methacrylate); polylactides; copolymers of L-glutamic acid and ethyl-L-glutamate; degradable lactic acid-glycolic acid copolymers; poly-D-(-)-3-hydroxybutyric acid; and other acrylic acid derivatives, such as homopolymers and copolymers of butylmethacrylate, methylmethacrylate, ethylmethacrylate, ethylacrylate, (2-dimethylaminoethyl)methacrylate, and (trimethylaminoethyl)methacrylate chloride.

If desired, a suitable therapy regime can combine administration of a prebiotic, probiotic and/or postbiotic composition of the disclosure with one or more additional therapeutic agents (*e.g.,* an inhibitor of TNF, an antibiotic, and the like). The peptide(s), other therapeutic agents, and/or antibiotic(s) can be administered, simultaneously, but may also be administered sequentially. Suitable antibiotics include aminoglycosides (*e.g.,* gentamicin), beta-lactams (*e.g.,* penicillins and cephalosporins), quinolones (*e.g.,* ciprofloxacin), and novobiocin. Generally, the antibiotic is administered in a bactericidal amount. A "bactericidal amount" is an amount sufficient to achieve a bacteria-killing concentration in the subject receiving the treatment. In accordance with its conventional definition, an "antibiotic," as used herein, is a chemical substance that, in dilute solutions, inhibits the growth of, or kills microorganisms. Also encompassed by this term are synthetic antibiotics (*e.g.,* analogs) known in the art.

Compositions provided herein can be used concurrently with other antibacterial agents including sulfa drugs such as sulfamethizole, sulfisoxazole, sulfamonomethoxine, sulfamethizole, salazosulfapyridine, silver sulfadiazine and the like; quinoline antibacterial agents such as nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like; antiphthisics such as isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like; antiacidfast bacterium drugs such as diaphenylsulfone, rifampicin and the like; antiviral drugs such as idoxuridine, acyclovir, vidarabine, ganciclovir and the like; anti-HIV agents such as zidovudine, didanosine, zalcitabine, indinavir sulfate ethanolate, ritonavir and the like; antispirocheteles; antibiotics such as tetracycline hydrochloride, ampicillin, piperacillin, gentamicin, dibekacin, kanendomycin, lividomycin, tobramycin, amikacin, fradiomycin, sisomycin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefinenoxime, cefpodoxime proxetil, cefpirome, cefozopran, cefepime, cefsulodin, cefinenoxime, cefinetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group and the like.

A composition (*e.g.,* a prebiotic, probiotic and/or postbiotic composition) provided herein can be combined with one or more steroidal drugs known in the art, including, but not limited to, aldosterone, beclometasone, betamethasone, deoxycorticosterone acetate, fludrocortisone acetate, hydrocortisone (cortisol), prednisolone, prednisone, methylprenisolone, dexamethasone, and triamcinolone.

A composition (*e.g.,* a prebiotic, probiotic and/or postbiotic composition) provided herein can be combined with one or more anti-fungal agents, including, but not limited to, amorolfine, amphotericin B, anidulafungin, bifonazole, butenafine, butoconazole, caspofungin, ciclopirox, clotrimazole, econazole, fenticonazole, filipin, fluconazole, isoconazole, itraconazole, ketoconazole, micafungin, miconazole, naftifine, natamycin, nystatin, oxyconazole, ravuconazole, posaconazole, rimocidin, sertaconazole, sulconazole, terbinafine, terconazole, tioconazole, and voriconazole.

For use in the therapeutic applications described herein, kits and articles of manufacture are also described herein. Such kits can comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers can be formed from a variety of materials such as glass or plastic.

For example, the container(s) can comprise one or more compositions (*e.g.,* a prebiotic, probiotic and/or postbiotic composition) provided herein, optionally in combination with another agent as disclosed herein. Such kits optionally comprise a composition disclosed herein with an identifying description or label or instructions relating to its use in the methods described herein.

The invention provides a more selective and potent method for killing and/or inhibiting the growth of *C*. *acnes.* The invention provides strains of skin commensal bacteria that produce selective antimicrobial activity against pathogenic bacterial strains, but not against other members of skin microflora. Therefore, antimicrobial therapy using commensal bacterial strains would selectively kill target strains of microorganisms. The capacity for selective killing of pathogenic bacteria over the normal microflora is highly desirable because it may help to maintain homeostasis and shape the normal bacterial community. Most skin commensal strains of CoNS produce multiple antimicrobials. In this case, antimicrobial therapy using commensal strains of bacteria would have a low risk of generating a resistant mutant against antibiotics. Because the strains of CoNS provided here are originally isolated from normal human skin, they would be low toxicity to the host. This invention may be used as a live bacterial application or as a sterile extract of the bacteria (*e.g.,* fermentation extract) or as purified active protein or as synthetic peptides.

The disclosure provides several coagulase-negative *Staphylococci* (CoNS) strains as well as two *Cutibacterium acnes* (*C. acnes*) strains that are potent antagonists of acne-associated *C*. *acnes* strains.

Table A provides a plurality of coagulase-negative *Staphylococci* (CoNS) strains that have anti-*P*. *acnes* activity:

**Table A:**

| Key# | Species | Strain/ Clone ID | Anti-*P.acnes* activity (ATCC6919)* | Anti-*P.acnes* activity (ATCC29322)* |
|---|---|---|---|---|
| 1 | *Staphylococcus epidermidis* | NIAMS009-G7 | ++ | ++ |
| 2 | *Staphylococcus epidermidis* | NIAMS018-F3 | +++ | +++ |
| *3* | *Staphylococcus warneri* | NIAMS025-G2 | + | + |
| 4 | *Staphylococcus epidermidis* | NIAMS028-H4 | - | - |
| 5 | *Staphylococcus epidermidis* | NIAMS028-C12 | - | - |
| 6 | *Staphylococcus lugdnensis* | NIAMS028-E7 | + | +++ |
| 7 | *Staphylococcus capitis* | NIAMS030-H8 | +++ | +++ |
| 8 | *Staphylococcus epidermidis* | NIAMS034-C1 | - | - |
| 9 | *Staphylococcus epidermidis* | AMT1-A9 | - | + |
| 10 | *Staphylococcus hominis* | AMT2-A12 | - | - |
| 11 | *Staphylococcus hominis* | AMT3-A12 | +++ | +++ |
| 12 | *Staphylococcus hominis* | AMT4-C2 | +++ | ++ |
| 13 | *Staphylococcus hominis* | AMT4-D12 | +++ | ++ |
| 14 | *Staphylococcus hominis* | AMT4-G1 | +++ | ++ |
| 15 | *Staphylococcus epidermidis* | AMT5-G6 | +++ | +++ |
| 16 | *Staphylococcus epidermidis* | AMT5-C5 | +++ | +++ |
| 17 | *Staphylococcus epidermidis* | S.epiA11 | - | - |
| 18 | *Staphylococcus hominis* | S.homC2 | +++ | ++ |
| 19 | *Staphylococcus epidermidis* | NIAMS037-H2 | - | - |
| 20 | *Staphylococcus epidermidis* | NIAMS037-A9 | - | - |
| 21 | *Staphylococcus epidermidis* | NIAMS038-A10 | - | - |
| 22 | *Staphylococcus epidermidis* | NLAMS038-F6 | +++ | +++ |
| 23 | *Staphylococcus hominis* | S.homA9 | ++ | - |
| 24 | *Stackvlococcus epidermidis* | S.epi1457 | - | - |

#17 in Table A (*S. epidermidis* A11) has been deposited with the ATCC as accession number PTO-125202; #23 in Table A (*S. hominis* A9) has been deposited with the ATCC as accession number PTA-125203; #24 in Table A was used as a negative control strain which produces no antimicrobial activity. See, Figure 4 for potency of antimicrobial activity against two *P. acnes* strains.

The disclosure exemplifies 4 strains of CoNS from skin: *S. capitis* N030_E12, *S. epidermidis* AMT5_C5, *S. epidermidis* N009_G7 and *S*. *epidermidis* N018_F3. A listing of sequences comprising all detected open reading frames from each of the foregoing strains accompanies this disclosure and is incorporated herein for all purposes. Accordingly, the strains of the disclosure (*e.g., S. capitis* N030_E12, *S. epidermidis* AMT5_C5, *S. epidermidis* N009_G7 and *S*. *epidermidis* N018_F3) can be identified using various probes and sequencing techniques and the sequence listing provided herein. For example, the strain *S*. *capitis* N030_E12 can be identified by determining whether an isolated *S*. *capitis* strain expresses one or more of the nucleic acid sequences identified by SEQ ID Nos: 1 to 2377 or produces PSMβ1, 3, 4 and 6 (see, *e.g.,* SEQ ID NOs: 9437, 9441, 9443 and 9447). Similarly, the strains *S*. *epidermidis* AMT5_C5, *S. epidermidis* N009_G7 and *S*. *epidermidis* N018_F3 can be identified by determining whether an isolated *S*. *epidermidis* AMT5_C5, *S. epidermidis* N009_G7 and *S*. *epidermidis* N018_F3 strain expresses one or more of the nucleic acid sequences identified by SEQ ID Nos: 2378 to 4765 (*S*. epi C5); 4766 to 7145 (*S*. epi, G7); or 7146 to 9435 (S. epi F3), respectively.

These strains are attractive candidates for biotherapy as they demonstrated broad antibacterial activity against multiple different strain types of *C*. *acnes* isolated from healthy and acne skin, but were less potent against other Gram-positive skin commensals and Gram-negatives **(Table 1).** In addition, two strains of *C*. *acnes: C. acnes_UCSD_*HI12 and *C*. *acnes_UCSD_HI30,* which demonstrated potent inhibition of different *C*. *acnes* strains by radial diffusion assay **(Table 2)** and in liquid culture **(****Fig. 3****)** are also described. The application/contacting of selected CoNS or *C*. *acnes* strains to the skin target and eliminant *C*. *acnes,* the etiological agent of acne, and not other skin resident bacteria which may play beneficial role in health.

**Table 1: Results of CoNS antagonism towards multiple different bacterial species by radial diffusion assay. Zone of killing/inhibition by radial diffusion: + small ++ medium +++ large**

| | **Rank order for application** | **1** | **2** | **3** | **4** |
|---|---|---|---|---|---|
| **Sequence type** | **Bacterial genus, species and strain name** | ***S. capitis* N030_E12** | ***S. epidermidis* AMT5_C5** | ***S. epidermidis* N009_G7** | ***S. epidermidis* N018F3** |
| N/A | *S. aureus* Newman | | +++ | ++ | +++ |
| N/A | *S. aureus* USA300 | | +++ | ++ | +++ |
| N/A | *S. aureus* 113 | +++ | +++ | +++ | +++ |
| N/A | Group A *Streptococcus* NZ131 | | | | |
| N/A | Group B *Streptococcus* DK23 | | | | |
| N/A | *S. hominus 27844* | + | + | + | |
| N/A | *S. lugdensis* | + | | | |
| N/A | *S. warneri* | | | | |
| N/A | *S. epidermidis 12228* | + | | | |
| N/A | *E. coli* RS218 | | | | |
| N/A | *P. aeruginosa* PA01 | | | | |
| N/A | *P. aeruginosa* PA4 | | | | |
| N/A | *S*. Typhimurium 14028 | | | | |
| N/A | *A. baumannii* AB5075 | | | | |

| **Sequence type** | ***Cutibacterium acnes* strain and site of origin** | ***S. capitis* N030_E12** | ***S. epidermidis* AMT5_C5** | ***S. epidermidis* N009_G7** | ***S. epidermidis* N018F3** |
|---|---|---|---|---|---|
| K1 | *C. acnes* HL042PA3 Health | +++ | ++ | ++ | +++ |
| K2 | *C. acnes* CRA7 Health | +++ | | | |
| K1 | *C. acnes* NIKH1 Health | ++ | + | + | + |
| A2 | *C. acnes* LNGA1 Health | +++ | ++ | ++ | ++ |
| H1 | *C. acnes* TRUF3 Health | +++ | | | |
| H1 | *C. acnes* A15NonLesional PA07 | +++ | ++ | + | ++ |
| C2 | *C. acnes* AI15NonLesional PA03 | ++ | +++ | +++ | +++ |
| E3 | *C. acnes* AI09NonLesional PA01 | +++ | +++ | +++ | ++ |
| E3 | *C. acnes* AI09NonLesional PA04 | +++ | ++ | ++ | ++ |
| F4 | *C. acnes* Lesional 104.1 | +++ | + | + | + |
| C2 | *C. acnes* AI01Lesional PA22 | +++ | ++ | ++ | +++ |
| A1 | *C. acnes* AI15Lesional PA10 | +++ | ++ | ++ | ++ |
| C2 | *C. acnes* AI01Lesional PA23 | +++ | ++ | + | + |

**Table 2: Several distinct C. acnes strains isolated from skin of healthy individuals (HI) were also found to inhibit the growth of different C. acnes strains from both healthy and acne-affected skin. Two distinct strains of C. acnes: C. acnes _UCSD_HI12 and C. acnes_UCSD_HI30 produced a potent inhibitory product against several strains of C. acnes. Both live cultures and sterile supernatant of both strains were found to inhibit the growth of C. acnes measured by radial diffusion in agar (Table 2) and liquid culture (Fig. 3).**

| ***C. acnes strains*** | | | ***C. a* 1** | ***C. a* 2** | ***C. a* 3** | ***C. a* 4** | ***C. a* 6** | ***C. acnes _UCSD_* HI12** | ***C.a*8** | ***C. a* 9** | ***C. a* 10** | ***C. a* 11** | ***C. acnes* _UCSD_ HI30** | ***C. a 12*** | ***C. a 13*** | **(control) *S. capitis*** |
|---|---|---|---|---|---|---|---|---|---|---|---|---|---|---|---|---|
| | **SLST** | **Origin** | **F4** | **F4** | **D1** | **D1** | **A1** | **D1** | **H1** | **K2** | **C1** | **C1** | **A5** | **F4** | **F1** | **N030_E12** |
| ***C. a* 30** | **A1** | **Health** | | | | | | | | | | | | | | **+++** |
| ***C*. *a* 31** | **A1** | **Health** | | | | | | | | | | | | | | **+++** |
| ***C. a* 32** | **H1** | **Acne** | | | | | | | | | | | **++** | | | **+++** |
| ***C. a* 33** | **H1** | **Acne** | | | | | | | | | | | **++** | | | **+++** |
| ***C*. *a* 34** | **K1** | **Health** | | | | | | **+** | | | | | | | | **+++** |
| ***C. a* 35** | **K1** | **Health** | | | | | | | | | | | | | | **++** |
| ***C. a* 36** | **K2** | **Health** | | | | | | | | | | | | | **+** | **++** |
| ***C*. *a* 37** | **K2** | **Health** | | | | | | **+++** | | | | | **+** | | | **++** |
| ***C*. *a* 38** | **C2** | **Acne** | | | | | | **+++** | | | | | **+** | | | **++** |
| ***C. a* 39** | **C2** | **Acne** | **+** | | | | | | | | | | **+** | | | **++** |
| ***C. a* 40** | **C1** | **Acne** | | | | | | | | | | | **+** | | | **+++** |
| ***C*. *a* 41** | **C1** | **Acne** | | | | | | | | | | | **++** | | | **+++** |
| ***C*. *a* 42** | **D1** | **Health** | | | | | | | | | | | | | | **+++** |
| ***C*. *a* 43** | **D1** | **Acne** | | | | | | | | | | | **++** | | | **+++** |
| ***C. a* 44** | **F4** | **Acne** | | | | | | **+** | | | | | | | | **+++** |
| ***C. a* 45** | **F1** | **Health** | | | | | | | | | | | **+** | | | **++** |
| ***S. aureus*** | **113** | **N/A** | | | | | | | | | | | | | | **+++** |
| ***S. capitis*** | **E12** | **N/A** | | | | | | | | | | | | | | |

The four *Staphylococcal* strains included in this disclosure have been sequenced and found to contain several biosynthetic gene clusters encoding predicted non-ribosomal peptide synthetases (NRPSs) and/or polyketide synthases (PKSs). These are major multi-modular enzyme complexes which synthesize secondary metabolites such as antimicrobial peptides, antibiotics and siderophores. Each module of an NRPS cluster activates a different amino or carboxyl acid, followed by their sequential condensation to synthesize a linear or cyclic natural product (see, **Fig. 1). Fig. 1** highlights such a NRPS cluster in *S*. *capitis* N030_E12 identified by antiSMASH - a comprehensive resource that permits the genome-wide identification, annotation and analysis of secondary metabolite biosynthetic gene clusters in bacterial genomes. The *S*. *capitis* NRPS cluster contains several genes with specific signature domains potentially involved in synthesis of an antimicrobial peptide such as gramicidin and tyrocidine. **Fig. 2** highlights a *trans*-AT polyketide synthase (PKS) cluster in *S*. *epidermidis* strains AMT5_C5, N009_G7 and N018_F3 identified by antiSMASH. The PKS machinery contains three essential domains: the acyl transferase (AT), the acyl carrier protein (ACP) and the ketosynthase (KS). For example, some polyketides produced by *Bacillus* species include difficidin and macrolactin. Therefore, this PKS cluster present within all three *S*. *epidermidis* species could be a source for novel antimicrobial peptides.

In one embodiment, the invention provides a probiotic composition for topical delivery comprising a CoNS commensal skin bacteria of the invention (*e.g., S. capitis* N030_E12, *S*. *epidermidis* AMT5_C5, *S. epidermidis* N009_G7 and/or S. *epidermidis* N018_F3). In a further embodiment, the topical composition contains only 1, 2, 3 or 4 of the strains selected from the group consisting of *S*. *capitis* N030_E12, *S. epidermidis* AMT5_C5, *S*. *epidermidis* N009_G7 and *S*. *epidermidis* N018_F3. In still another embodiment, a topical probiotic composition of the disclosure can comprise or consist of a commensal skin bacteria selected from the group consisting of *S*. *capitis* N030_E12, *S. epidermidis* AMT5_C5, S. *epidermidis* N009_G7 and S. *epidermidis* N018_F3, and any combination of the foregoing, wherein the commensal skin bacteria is substantially pure (*e.g.,* 80%, 85%, 87%, 90%, 92%, 95%, 98% or 100% pure of other bacteria species found on the skin of a subject.

In some embodiments, the composition comprises a cream, ointment, oil suspension or unguent wherein the probiotic bacteria (*e.g., S. capitis* N030_E12, *S. epidermidis* AMT5_C5, *S. epidermidis* N009_G7, *S. lugdnensis* N028_E7, and/or *S*. *epidermidis* N018_F3) as described above are incorporated within a moisturizer or emulsion such as those described below and in Nakatsuji, T. et al. (2016), Nature Medicine. In some embodiments, the composition comprises a patch or poultice wherein the bacteria are combined with a suitable excipient and are incorporated within a fabric, gel matrix, or polymer sheet. Suitable excipients and carriers for topical administration are known in the art and include thickeners, emulsifiers, fatty acids, polysaccharides, polyols, and polymers and copolymers, including, without limitation, alginate, microcrystalline cellulose, polylactic acid, polylactic-co-glycolic acid, petrolatum, and numerous others known in the art.

In some embodiments, the composition comprises a bacterial culture medium, a conditioned bacterial culture medium, and/or a bacterial culture. In some embodiments, the composition comprises a filtrate or supernatant of a bacterial culture medium. In some embodiments, the composition comprises a lyophilized culture medium. In some embodiments, the composition comprises a lyophilized conditioned culture medium produced from a filtrate or supernatant of a bacterial culture medium.

In some embodiments, the method as described herein comprises supporting the health of the skin of a subject. In further embodiments, the method comprises providing a treatment for skin dysbiosis and disorders (*e.g.,* acne) derived therefrom. In some embodiments the method comprises providing a treatment for bacterial infection of the skin (*e.g.,* C. acne infection or overgrowth). In the disclosure, the treatment may comprise the steps of: identifying a subject with skin dysbiosis, bacterial infection, acne; and administering to the site of the condition in need of treatment the probiotic compositions as disclosed herein. Determination of the appropriate mode of administration of a given formulation (ointment, gel, patch, etc.) can be done by one of ordinary skill in the art of treating skin infections. The probiotic compositions may be re-applied at regularly timed intervals. The probiotic compositions may be reapplied every three days. The probiotic compositions may be reapplied every two days. The probiotic compositions may be reapplied every two days. The probiotic compositions may be reapplied daily. The probiotic compositions may be reapplied more than once per day. The probiotic compositions may be reapplied weekly. The probiotic compositions may be only applied a single time.

Tthe method of the disclosure comprises providing a treatment for *C*. *acnes* infections. The method comprises the steps of: diagnosing a *C*. *acnes* infection; and applying to the site of the infection the probiotic and/or prebiotic compositions as disclosed herein, wherein such compositions are capable of killing or inhibiting the growth of *C*. *acnes,* either by production of antimicrobial compounds, by competition for resources within the cutaneous or mucosal biota, or by other means. Determination of the appropriate mode of administration of a given formulation (ointment, gel, patch, etc.) can be done by one of ordinary skill in the art of treating skin infections. The probiotic compositions may be re-applied at regularly timed intervals (*e.g.,* daily, every two days, every three days, weekly, etc.). It will be apparent to one of ordinary skill in the art that similar or identical steps can be applied to provide a treatment for *C*. *acnes.* The method may comprise providing a treatment for infections with unknown or uncharacterized pathogens. The method may comprise providing a treatment for a chronic skin condition.

A commensal bacterial of the invention can be isolated from human skin and identified using methods described herein. For example, the disclosure provides a method of obtaining, identifying and culturing a commensal bacteria described herein by swabbing human skin surface using, *e.g.,* a foam tip swab. The swabs were placed in tryptic soy broth. The broth is diluted onto mannitol salt agar plates (MSA) supplemented with 3% egg yolk. Pink colonies without halo representing coagulase-negative *Staphylococci* (CoNS) strains are collected and grown in tryptic soy broth (TSB). The strain can then be assayed as described in **Fig. 4** (see also, **Fig. 5A****).** Strains with strong inhibition of *C*. *acnes* can be further characterized by DNA isolation and sequencing or by PCR, southern or northern blots. In addition, measurement of the expression profile of phenol soluble modulins can also be measured. In some embodiments, organisms expressing PSMβ1, 3, 4 and 6 (*e.g.,* SEQ ID NOs: 9437, 9441, 9443, and 9447) can be identified as S. *capitis.* PSMβ from *S*. *epidermidis* are provided in SEQ ID NOs: 9449, 9451 and 9453.

gDNA can be isolated using any number of commercially available kits (*e.g.,* DNeasy UltraClean Microbial Kit, Qiagen). The gDNA can be sequenced using various sequence platforms (*e.g.,* MiSeq; Illumina Inc., San Diego, CA for two cycles, which can generated 2x 250 bp paired-end reads). Adapters are removed using cutadapt (see, *e.g.,* world-wide-web at cutadapt.readthedocs.io/en/stable/). Low-quality sequences can be removed using Trim Galore (see, *e.g.,* world-wide-web at bioinformatics.babraham.ac.uk/projects/trim_galore/) with default parameters. Sequences mapping to the human genome are removed from the quality-trimmed dataset using the Bowtie2 program (ver. 2.28) (I) with parameters (-D 20 -R 3 -N 1 -L 20 - very-sensitive-local) and the human reference genome hg19. The filtered reads are *de novo* assembled using SPAdes (version 3.8.0) with k-mer length ranging from 33-127. The genome is annotated with rapid annotation of microbial genomes using subsystems technology (RASY) with default parameters. Predicted amino acid sequences and or the nucleic acid sequences can be compared to the sequence listing attached hereto to identify *S*. *capitis* N030_E12, *S. epidermidis* AMT5_C5, *S. epidermidis* N009_G7 and S. *epidermidis* N018_F3.

The invention also provides compositions that comprise at least 2, at least 3, at least 4, at least 5 or at least 6 PSMβ. In some embodiments, the composition comprises at least 4 PSMβ. In yet other embodiments the composition comprises at least 5 PSMβ. In any of the foregoing embodiments, the PSMβ are selected from PSMβ1, 2, 3, 4, 5, and 6. In certain embodiments, the composition comprises at least PSMβ1, 3, 4, and 6.

The invention also provides formulations for topical administration comprising comprise at least 2 PSMβ. In some embodiments, the formulation comprises at least 3 PSMβ. In yet other embodiments the formulation comprises at least 4 PSMβ. In any of the foregoing embodiments, the PSMβ are selected from PSMβ1, 2, 3, 4, 5, and 6 produced from *S*. *capitis* E12 and/or having a sequence that is at least 85%, 90%, 92%, 94%, 95%, 96%, 97%, 98%, 99% or 100% identical to the PSMs set forth in each of SEQ ID NOs: 9437, 9439, 9441, 9443, 9445, 9447. In certain embodiments, the formulation comprises at least PSMβ1, 3, 4, and 6. In some embodiments, the formulation comprises at least 3 of PSMβ1, 3, 4, and 6 and a probiotic, prebiotic or postbiotic of the invention.

The disclosure also provides a method of diagnosing skin dysbiosis and/or risk of a *C*. *acnes* infection. The method comprises obtaining a biological sample from the skin of a subject and determining the relative abundance of commensal bacteria in the sample, wherein the abundance includes determining the abundance of at least the presence of *S*. *capitis* and *S*. *epidermidis.* More particularly, the determining can measure the abundance of at least 1 to 4 (*e.g.,* 1, 2, 3 or 4) of the bacteria selected from the group consisting of *S*. *capitis* N030_E12, *S. epidermidis* AMT5_C5, *S*. *epidermidis* N009_G7, and *S*. *epidermidis* N018_F3. If the level or abundance of S. capitis or *S*. epidermidis (*e.g., S. capitis* N030_E12, *S. epidermidis* AMT5_C5, *S. epidermidis* N009_G7, *S*. *epidermidis* N018_F3) falls below a threshold of less than 50%, 40%, 30%, 20%, 10%, 5%, 2.5%, 1%, 0.5%, 0.1% then the subject is at risk of having a C. acnes infection.

The presence of one or more the PSMβ produced by *S*. *capitis* E12 can be determine in a sample from the subject in determining if the subject has or is at risk of having a C. *acnes* infection. For example, the sample can be obtained from the skin of a subject, extracted with n-butanol and the presence of PSMβs measured as described herein. The presence of PSMβ1, 3, 4 and 6 in the sample is indicative of a reduced risk of infection. In contrast no or low levels (levels below a normal average control) is indicative of a subject having or at risk of having a *C*. *acnes* infection. A "normal" or "average control" level is a level of PSMβ (*e.g.,* PSMβ1, 3, 4, and/or 6) that is the mean level of total PSMβ or mean level of each type of PSMβ (*e.g.,* PSMβ1, 3, 4 or 6) in a population of subjects that do not have a *C*. *acnes* infection. In one embodiment a determination of the sequence of each PSMβ present in the extract sample can be determined and then compared to the sequences as set forth in SEQ ID NOs: 9437, 9439, 9441, 9443, 9445, 9447, to determine which type of PSMβ is present.

The methods of diagnosis and/or prognosis of the disclosure can be used to determine if a subject needs a prebiotic, probiotic or postbiotic therapy as described herein.

The following examples are exemplary and are not intended to limit the claimed invention, but rather illustrate methods of compositions of the disclosure.

### EXAMPLE

**Human subjects and sample swab collection.** All experiments involving human subjects were carried out according to protocols approved by the University of California, San Diego (UCSD) IRB (Project#140144). Swab sample collection was performed on 12 healthy adults with swabs taken from the upper arm and the face. Swabs were incubated in 3% Tryptic Soy Broth (TSB), vortexed briefly and plated onto mannitol salt agar containing egg yolk (MAY) plate for selective growth of staphylococcal species. *S*. *aureus* were distinguished from CoNS according to mannitol metabolism and the egg yolk reaction. *C. acnes* clinical isolates were obtained by swabbing lesional and non-lesional facial skin sites of 10 acne patients as well as facial skin from 6 healthy volunteers. Swabs were incubated in reinforced clostridial media (RCM), vortexed and plated onto brucella blood agar plates supplemented with Vitamin K, hemin and 5% sheep's blood and incubated for 5 days at 37°C.

**Screening for antimicrobial activity from skin-derived CoNS.** 24 individual isolated colonies of CoNS, that were selected from each skin site, were randomly picked from a MAY plate and transferred to TSB (1 mL) in a deep 96 well plate. Each plate contained a previously characterized CoNS strain, which included *S*. *epidermidis* ATCC1457 that failed to demonstrate antimicrobial activity against other staphylococci (negative control) as well as *S. hominus* A9 which was previously demonstrated to produce lantibiotics that kill S. *aureus* (positive control). The CoNS plate was sealed with sterile Aeraseal film (sigma) and cultured at 37°C overnight, shaking at 250 rpm. Bacterial growth was evaluated by measuring OD₆₀₀ and only CoNS grown to a high density (OD₆₀₀>6.0) were used for subsequent analysis. To measure antimicrobial activity in the secreted supernatant, the CoNS supernatant from overnight culture was harvested and sterile filtered. To measure antimicrobial activity from live growing CoNS, bacteria from overnight culture were pelleted by centrifugation then the supernatant was discarded, and the bacteria were resuspended in fresh TSB.

**Mass spectrometry.** A portion of the fractions of interest (< 1 µg) were dried under vacuum and resuspended in 5 µL of 5% acetonitrile with 5% formic acid. Next, individual LC-MS experiments were conducted on 1/5 of each sample through 85 minutes of data acquisition on an Orbitrap Fusion (Thermo Fisher Scientific) mass spectrometer with an in-line Easy-nLC 1000 (Thermo Fisher Scientific). A home-pulled and packed 30 cm column was triple-packed with 0.5 cm, 0.5 cm and 30 cm of 5 µm C4, 3 µm C18, and 1.8 µm C18 respectively and heated to 60 °C for use as the analytical column. Peptides were first loaded at 500 bar which was followed by a chromatography gradient ranging from 6 to 25% acetonitrile over 70 minutes followed by a 5-minute gradient to 100% acetonitrile, which was held for 10 minutes. Electrospray ionization was performed by applying 2000V through a stainless-steel T-junction connecting the analytical column and Easy-nLC system. Each sample was followed by two washes starting with a gradient from 3 to 100% acetonitrile over 15 minutes with an additional 10 minutes at 100% acetonitrile. An m/z range of 375-1500 was scanned for peptides with charge states between 2-6. Centroided data was used for quantitation of peaks. Acquisition was run in a data-dependent positive ion mode. Raw spectra was searched in Proteome Discoverer Version 2.1 against a uniprot reference database for *Staphylococcus capitis* (Uniprot proteome UP000042965, accessed 10/01/2018) using the Sequest algorithm alongside a reverse database approach used to control peptide and protein false discoveries to 1%. No enzyme was specified in the search and a minimum peptide length was set to 6 amino acids. Search parameters included a precursor mass tolerance of 50 ppm and fragment mass tolerance of 0.6 Da and variable oxidation for modifications.

**In *vitro* antimicrobial assays.** For the initial CoNS antimicrobial screen, the radial diffusion agar assays were performed using S. *aureus* 113 or either *C*. *acnes* HL110PA3 (health-associated, SLST K1) or *C*. *acnes* HL096PA1 (acne-associated, SLST C2). For the radial diffusion agar assays, melted TSB or RCM agar (12 mL) was mixed with *S*. *aureus* or *C*. *acnes* (1x10⁶ CFU) and poured into a 10 cm square petri square. When the agar was solidified, a 10 µl aliquot of bacteria was inoculated onto a single grid. The plates were incubated at 37°C overnight shaking (for *S*. *aureus*) or incubated at 37°C for 3 days in a 2.5L anaeroPack (thermo Scientific) (for *C*. *acnes*) to allow visible growth of bacteria. Antibacterial activity was indicated by a clear zone of inhibition within the agar that surrounds the colony. The size of the inhibitory zone was recorded as a measure of antimicrobial activity (+ slight, ++ moderate, or +++ potent). For the liquid culture assay, conditioned supernatant from overnight cultures of CoNS were harvested and sterile filtered (0.22 µm). 50% of conditioned supernatant was mixed with 50% fresh TSB or RCM containing 1X10⁵ CFU/mL of *S*. *aureus* or *C*. *acnes,* in a 96 well round bottom plate. *S*. aureus plates were incubated at 37°C shaking overnight and *C*. *acnes* plates incubated standing at 37°C in an anaerobic chamber. Bacterial growth was measured by OD₆₀₀ and positive antimicrobial strains were identified as those that suppressed bacterial growth to less than 50% (I₅₀) of growth measured for negative control strains. Bacterial survival was measured by counting the number of CFU on TSB plates (*S. aureus*) or Brucella blood agar plates (*C*. *acnes*)*.* At selected times post-treatment with bacterial supernatant or extracts, the number of CFU was determined by serial dilution in phosphate-buffered saline (PBS) and plating onto appropriate agar media. Bacterial survival was measured as the total number of CFU per milliliter.

**Purification of antimicrobials produced by CoNS strains.** Conditioned media from overnight cultures of the selected antibacterial *S*. *capitis* E12 strain, including positive control antimicrobial *S*. *hominus* A9 strain, were first sterilized by filtration through a 0.22µm Millipore filter. Initial characterization involved treating the sterile bacterial supernatant to boiling at 100°C for 15 mins or incubation with proteolytic enzymes papain and proteinase K, at 200 µg/mL at 37°C for 60 mins. Antimicrobial activity was measured by radial diffusion assay. For size exclusion of the antimicrobial molecule, 20 mL of conditioned media was loaded onto a 3, 10 and 30 kDa molecular weight cutoff (MWCO) columns (Pierce) and centrifuged at 4000 x g for 15 mins. Whilst the flow-through fraction was set aside, and the retained fraction was washed 2X in PBS and resuspended with 20 mL TSB. Antimicrobial activity was assessed for both fractions. To precipitate the active molecule, ammonium sulphate was added to the sterile supernatant (30-80% saturation) for 1 hour under constant rotation at room temperature, followed by centrifugation at 4000 x g for 45 mins. The resulting pellet was washed 3X with H₂O and reconstituted in H₂O. Antimicrobial activity was determined by *S*. *aureus* and *C*. *acnes* growth in agar and liquid culture.

**Solid Phase Extraction (SPE) and HPLC purification of supernatant.** *S. capitis* E12 sterile supernatant that had been retained on a 10 kDa MWCO column and precipitated in 60% AS was applied on an Oasis HLB cartridge (Waters). The cartridge was washed in 20% acetonitrile in H₂O and eluted in 80% acetonitrile in H₂O. The eluted fractions were lyophilized and reconstituted in H₂O. The 80% fraction was then loaded onto a C8 Sep-Pak cartridge (Waters), washed in 20% acetonitrile and eluted in 50% acetonitrile. The fractions were lyophilized, reconstituted in H₂O and then assessed for antimicrobial activity and visualized by silver stain (Pierce) according to manufacturer's instructions. First step HPLC purification was carried out with 1 mg of S. capitis E12 butanol extract loaded into a CapCel Pak C8 (5 µm, 300 Å, 4.6 × 250mm) (Shiseido Co.) with a linear gradient of acetonitrile from 10% to 60% in 0·1% (v/v) TFA at 0.8 mL/min. Fractions were lyophilized then resuspended in H₂O and antimicrobial activity assessed by liquid culture assay. Up to five sequential purifications were carried out with each single antimicrobial fraction pooled together for second step HPLC. For the second purification, a linear gradient of acetonitrile from 25% to 50% was adopted.

**Transplantation of antimicrobial CoNS on pigskin and mice.** All experiments involving live animal work were in accordance with the approval of the Institutional Animal Care and Use Guidelines of the University of California, San Diego. Fresh-frozen pig skin sheets were obtained from Loretta Tomlin Animal Technologies (Livermore, CA) and sanitized by surgical brush with 3% chloroxylenol. The skin sheet was cut into 2.5cm × 2.5cm and rinsed 20X times with sterile PBS more. 1x10⁷ CFU *C*. *acnes* (ATCC6919) was epicutaneously challenged on pig skin for 1 hour and 100 µl of *S*. *capitis* E12 extract (10 mg/mL) or PBS control was applied for 24 h. Live bacteria were harvested by swabbing to measure *C*. *acnes* survival. For mouse experiments, the backs of hairless SKH1 mice were scrubbed with alcohol wipes and 1x10⁷ CFU *C. acnes* was inoculated onto sterile gauze pads which were placed onto the dorsal skin and secured with tegaderm film for 24 h. *C*. acnes-containing gauze pad was removed and 100 µl of *S*. *capitis* E12 extract (10 mg/mL) or PBS control was inoculated onto fresh gauze pad and placed back onto the same dorsal skin site and secured with tegaderm film for an additional 24 h. 48 h post-inoculation, skin was swabbed to measure surviving CFU of *C. acnes.*

**Screening for antimicrobial activity from human skin bacteria identifies a *S. capitis* strain with potent and selective activity against *C*. *acnes.*** A library of 288 CoNS isolates were collected from skin swabs of the forearm and face of healthy volunteers. An unbiased analysis of anti-*C. acnes* activity was performed by live coculture with the CoNS isolate growing on agar with *C*. *acnes* and measuring the growth of *C*. *acnes* following the addition of 50% v/v conditioned CoNS supernatant that was sterile filtered **(****Fig.5A****).** Results of the functional screen revealed a total of 13 isolates that were active against the growth of *C*. *acnes* in both the liquid assays and agar coculture **(****Fig.5B,C****).** DNA extracted from the CoNS isolates with antimicrobial activity was subjected to 16S sequencing and several different species were identified, including *S*. *epidermidis* and *S*. *hominis* which are considered frequent colonizers of human skin. The most potent isolate was identified as *Staphylococcus capitis* (*S. capitis* strain E12) and was used for further characterization. Of the 13 antimicrobial strains identified, all were isolated from the forearm. None of the facial isolates were found to inhibit the growth of *C. acnes.* Interestingly, the lantibiotic-producing *S. hominus* A9 strain that is active against *S. aureus* was ineffective against *C. acnes,* illustrating the selective nature of antimicrobials derived from the mixed bacterial community of the skin.

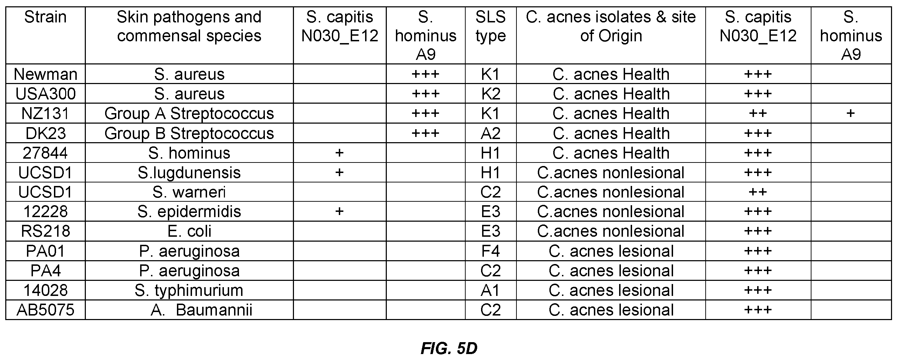

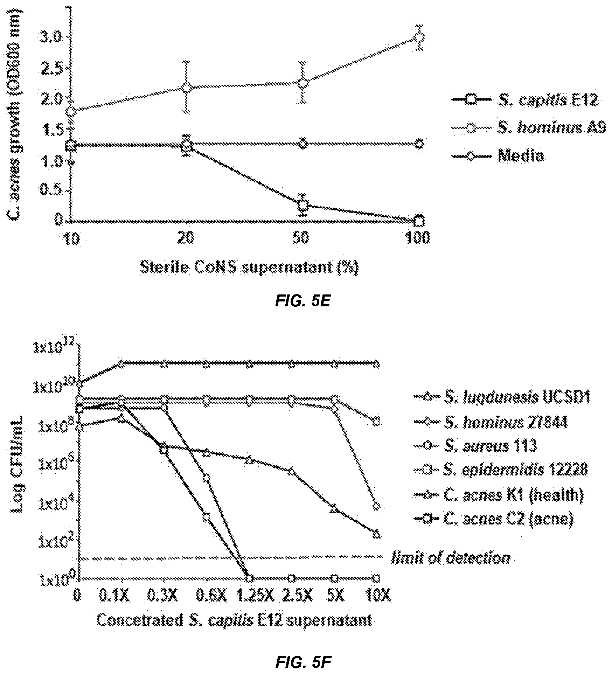

Next, the selectivity of *S. capitis* E12 to inhibit the growth of several different CoNS commensal species and common skin pathogens was tested by agar assay. Whilst the *S. hominus* A9 potently inhibited the growth of *S. aureus* and GAS, *S. capitis* E12 exhibited only weak activity against CoNS and was ineffective against other skin pathogens **(****Fig.5D****).** Strikingly, *S. capitis* E12 exhibited potent activity against a wide range of *C. acnes* strains, including strains that were isolated from either lesional or non-lesional skin of acne patients. 50% dilution of unconcentrated media sterile supernatant from *S. capitis* E12 was sufficient to inhibit *C. acnes* growth, thus validating the findings from the functional screen and demonstrating that under normal growth conditions this strain of *S. capitis* can produce sufficient antimicrobial activity to outcompete *C. acnes* (**Fig. 5E**). To investigate if the antimicrobial supernatant is bactericidal, the number of surviving *C. acnes* colonies was enumerated after 24 h treatment with increasing concentrations of *S. capitis* E12. Consistent with results from the functional screen, S. *capitis* supernatant was generally not bactericidal against other CoNS species, with exception to the *S. hominus* strain 27844 only at the highest 10X concentration. However, the *S. capitis* E12 supernatant was bactericidal against *C. acnes,* showing a 6-log decrease of *C. acnes* during exposure to 0.6X (60%) supernatant and complete sterilization of the culture at highest concentrations (**Fig. 5F**).

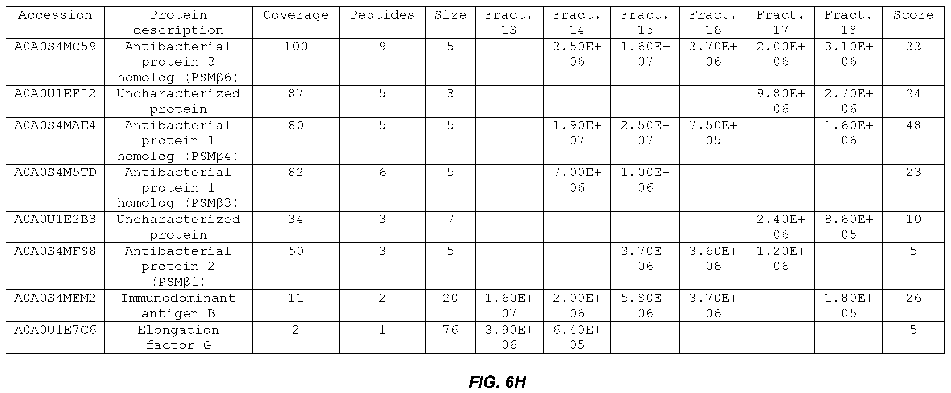

**Purification and identification of PSMβ antimicrobial peptides.** Initial examination into the nature of the antimicrobial factor revealed it to be resistant to heat treatment, but sensitive to proteolysis, suggestive of a proteinaceous molecule (Suppl. Fig.1A,B). Furthermore, the antimicrobial factor was precipitated from supernatant during incubation with 60-80% ammonium sulphate (AS) and concentrated by centrifugation **(****Fig.9C****).** Next, sample preparation was carried out on the concentrated AS precipitate using solid phase extraction. Samples were first loaded onto a hydrophilic-lipophilic balanced (HLB) column and the *anti-C. acnes* molecule was found to be eluted at 80% acetonitrile, as determined 72 h after inoculation onto *C. acnes* agar **(****Fig.6A****)** or with *C. acnes* in liquid culture **(****Fig.6B****).** The active elution was subsequently loaded onto a hydrophobic C8 cartridge and the anti-*C. acnes* molecule was eluted at 50% acetonitrile **(****Fig. 6A,B****).** Total protein staining of the fractions revealed a highly enriched band(s) of roughly 4-5 kDa in size, with greater purity visible during subsequent elution steps **(****Fig. 5C****).** Reverse phase high performance liquid chromatography (HPLC) revealed several peaks, of which a single fraction (fraction 21) was found to suppress the growth of *C. acnes* in liquid culture **(****Fig. 5D****).** To ensure the highest purity, the active fractions were then pooled together from four separate HPLC runs and a second step HPLC purification was performed. This time, several small peaks were visualized **(****Fig. 6F****),** and two fractions (fractions 15 and 16) were found to suppress the growth of *C. acnes* in liquid culture **(****Fig. 6G****).** Mass spectrometry (MS) analysis of active fractions (15,16) and control non-active fractions (13,14,17,18) revealed four candidate peptides referred to as "antibacterial protein" each 5 kDa in size (Fig. 6H). BLAST search of the peptide sequences identified these as belonging to the beta class family of phenol soluble modulins (PSMβ) present within multiple Staphylococcal species.

Whilst 4 distinct PSMβ peptides were detected by MS, analysis of the *S. capitis* E12 genome revealed up to 6 PSMβ-encoding genes, contained within an operon-like structure **(****Fig. 7A****).** All six PSMβ-encoding genes are closely related, with amino acid sequence identity ranging from 56% to 91%, and predicted charge ranging from -1 to +1 **(****Fig. 7B****).** Further analyses found these genes to be absent from genomes of ATCC strains of *S. capitis* 35661 and 27844 and indeed no antimicrobial activity was detected against *C. acnes* from these laboratory strains **(****Fig. 7C****).** Alpha helical wheel plots (Heliquest) also predicted these peptides to form an α-helix, amphipathic-like structure for each PSMβ, with separate hydrophobic and hydrophilic faces on the opposite sides of the long axis of the peptide. This amphipathic structure is common amongst many well characterized antimicrobial peptides **(****Fig. 7D****).**

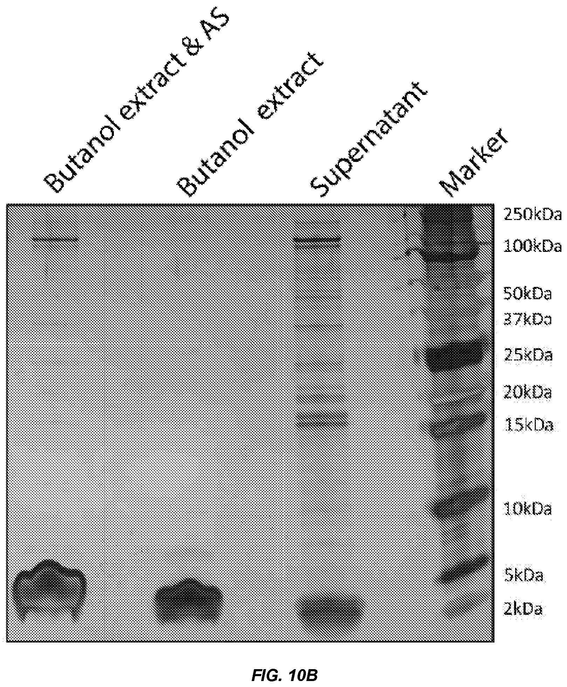

**PSMβ inhibits skin colonization by *C*.** ***acnes.** S. capitis* E12 conditioned supernatant was extracted with n-Butanol, a procedure that enriches for PSM peptides. This resulted in a relatively pure extract enriched for all PSMβ peptides, and retained activity against *C*. *acnes* **(****Fig. 10**). To confirm that PSMβ peptides were active as the anti-*C*. *acnes* molecule, and identify which peptide had opitimal activity, PSMβ1 and 6, the most abundant molecules from the purified active fractions (15 and 16, **Fig 6H****),** as well as PSMβ4, which was less abundant, were individually synthesized. All three PSMs were shown to possess antimicrobial activity *in vitro* **(****Fig. 8A****).** PSMβ1 and 6 both effectively inhibited growth of *C. acnes* at 62 µg/ml, whilst PSMβ4 was less potent, inhibiting at 125 µg/ml. Interestingly, the combination of all three peptides exhibited optimal activity against *C. acnes,* inhibiting growth in 31 µg/ml. However, this was not as inhibitory compared to the same concentration of the butanol extract, which inhibited at 8 µg/ml. This discrepancy could be due to the presence of the additional fourth PSMβ peptide (PSMβ3) found within the extract. Several bacterial antimicrobials have been shown to synergize with human antimicrobials such as defensins and LL37. However, exposure of *C. acnes* to *S. capitis* extract with or without LL-37 peptide did not result in enhanced killing activity **(****Fig. 11****).** Given the greater potency and relative purity of the PSMβ-containing extract compared to the synthetically produced PSMβ peptides, the extract was used in subsequent experiments to determine the efficacy and potency of *S. capitis* E12 *in vitro* and *in vivo* experiments.

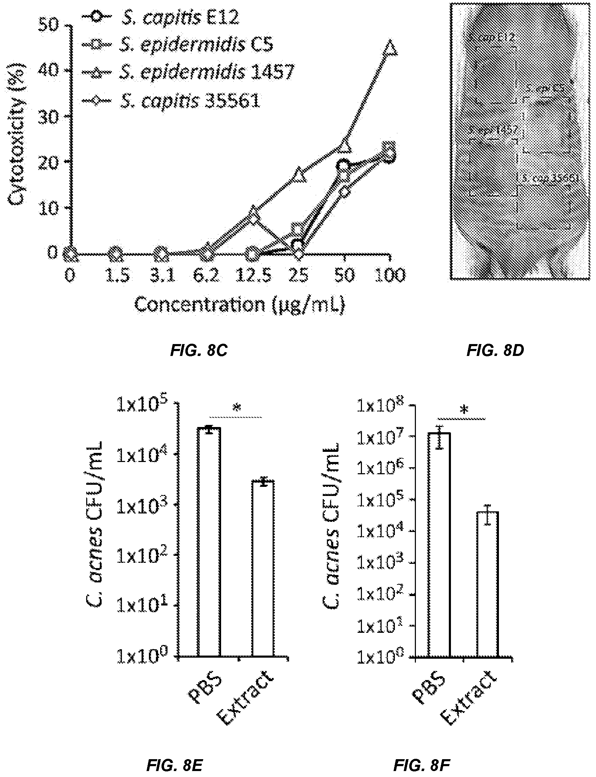

*S. capitis* E12 extract was next compared to several antibiotics commonly used for acne treatment. The extract was more potent against *C. acnes* than erythromycin, clindamycin and tetracycline, antibiotics which are commonly prescribed for acne treatment **(****Fig. 8B****).** Moreover, no increase in the MIC (≤16 µg/mL) was recorded for *C. acnes* during treatment with the extract for up to 20 generations, indicating relative inability to promote resistance. Furthermore, exposure of primary human keratinocytes (NHEKs) to extracts did not produce a significant cytotoxic response from these epidermal cells as measured by lactate dehydrogenase (LDH) release **(****Fig. 8C****).** Likewise, mouse back skin exposed to different strains of CoNS for 24 h showed that S. *capitis* E12 did not induce observable erythema or injury **(****Fig. 8D****).** Finally, having confirmed the expected result that this commensal skin bacterium was well tolerated by the epidermis, *C. acnes* was epicutaneously applied to colonize pig skin *ex vivo* or back skin of live SKH1 mouse, and then extract from *S. capitis* E12 was applied. A single treatment resulted in a significant reduction in C. *acnes* CFU in both models **(****Fig. 8E,F****).** Overall, these results demonstrate PSMβ from *S. capitis* E12 has capacity to kill *C. acnes* and illustrates the potential of exploiting naturally occurring microbial events in the human skin for development of therapeutics.

## Claims

1. A topical probiotic composition comprising one or more bacterial strains for use in treating a dermatological disease or disorder associated with *Cutibacterium acnes* (*C. acnes*), wherein the one or more bacterial strains comprises *Staphylococcus capitis* (*S. capitis*) N030_E12, *Staphylococcus epidermidis* (*S. epidermidis*) N009_G7, *S. epidermidis* N018_F3, or any combination thereof, and
wherein at least one of the one or more bacterial strains is capable of expressing a peptide comprising a sequence selected from the group consisting of: MTKLAEAIANTVKAGQDHDWAKLGTSIVGIAENGIGLLGKVFGF (SEQ ID NO: 9437), MTKLAEAIANTVKAGQDHDWAKLGTSIVGIAENGIGALSKIFGG (SEQ ID NO: 9439), MQKLAEAIANTVKAGQDHDWAKLGTSIVGIAENGINAITKIFGG (SEQ ID NO: 9441), MTKLAEAIANAVKAGQDQDWAKLGTSIVGIAENGISLLGKVFGF (SEQ ID NO: 9443), MQKLAEAIANTVKAGQDHDWTKLGTSIVDIVENGVSALTKVFGG (SEQ ID NO: 9445), and MEKLFDAIRNTVDAGINQDWTKLGTSIVDIVDNGVKVISKFIGA (SEQ ID NO: 9447).

2. The topical probiotic composition for use according to claim 1, wherein the topical probiotic composition is formulated for the topical treatment of disorders of dysbiosis of the skin, scalp, or mucosae;
wherein the topical probiotic composition inhibits the growth of *C. acnes;* and
wherein the one or more bacterial strains is provided in a live form.

3. The topical probiotic composition for use according to any one of claims 1-2, wherein the topical probiotic composition is formulated for the topical treatment of disorders of dysbiosis of the skin, scalp, or mucosae;
wherein the topical probiotic composition inhibits the growth of *C. acnes;* and
wherein the one or more bacterial strains is provided in a lyophilized or freeze-dried or spray-dried form.

4. The topical probiotic composition for use according to claim 3, wherein the one or more bacterial strains can be reconstituted into a live form.

5. The topical probiotic composition for use according to any one of claims 1-4, wherein the topical probiotic composition comprises a thickened topical formulation of the one or more bacterial strains;
wherein the topical probiotic composition is formulated for the topical treatment of disorders of dysbiosis of the skin, scalp, or mucosae;
wherein the topical probiotic composition inhibits the growth of *C. acnes;* and optionally,
wherein the composition comprises a prebiotic compound, a protectant, humectant, emollient, abrasive, salt, and/or surfactant.

6. The topical probiotic composition for use according to any one of claims 1-5, wherein the topical probiotic composition is formulated for the treatment of disorders of dysbiosis of the skin, scalp, or mucosae, and/or wherein the composition is formulated as a cream, ointment, unguent, spray, powder, oil, thickened formulation, or poultice.

7. The topical probiotic composition for use according to any one of claims 1-6, wherein the composition inhibits C. acnes growth, viability, or activity.

8. The topical probiotic composition for use according to any one of claims 1-7, wherein the dermatological disease or disorder is acne, chronic blepharitis, and/or endophthalmitis.

9. A pharmaceutical composition comprising a plurality of phenol-soluble modulin peptides (PSM), comprising PSMβ1 (SEQ ID NO: 9437), PSMβ3 (SEQ ID NO: 9441), PSMβ4 (SEQ ID NO: 9443), and PSMβ6 (SEQ ID NO: 9447); optionally, the plurality of phenol-soluble modulin peptides (PSM) comprises PSMβ1 (SEQ ID NO: 9437), PSMβ2 (SEQ ID NO: 9439), PSMβ3 (SEQ ID NO: 9441), PSMβ4 (SEQ ID NO: 9443), PSMβ5 (SEQ ID NO: 9445) and PSMβ6 (SEQ ID NO: 9447); and further optionally, a pharmaceutically acceptable carrier.

10. The pharmaceutical composition according to claim 9, wherein the pharmaceutical composition comprises one or more bacteria selected from the group consisting of *S. capitis* N030_E12, *S. epidermidis* N009_G7, and *S. epidermidis* N018_F3.

11. The pharmaceutical composition according to any one of claims 9-10, comprising a cell-free fermentation extract obtained from a culture of *S. capitis* N030_E12, *S. epidermidis* N009_G7, *S. epidermidis* N018_F3, or any combination thereof.

12. The pharmaceutical composition of any one of claims 9-11 for use in treating a dermatological disorder associated with *C. acnes,* skin or mucosal infections, atopic dermatitis, psoriasis, mastitis, acne, chronic blepharitis and endophthalmitis, or other disorders related to skin dysbiosis in humans or other mammals, wherein the composition is for application to the skin or mucosa in an effective amount of the pharmaceutical composition to a subject in need thereof.

13. The pharmaceutical composition for use according to claim 12, wherein the pharmaceutical composition is for application topically, and wherein the pharmaceutical composition is formulated as a cream, ointment, unguent, spray, powder, oil, a lotion, tincture, thickened formulation, or poultice.

14. The pharmaceutical composition for use according to any one of claims 12-13, wherein the pharmaceutical composition inhibits *C. acnes* growth, viability, or activity.

## Patentansprüche

1. Topische probiotische Zusammensetzung, umfassend einen oder mehrere Bakterienstämme zur Verwendung bei der Behandlung einer dermatologischen Erkrankung oder Funktionsstörung im Zusammenhang mit *Cutibacterium acnes* (*C. acnes),* wobei der eine oder die mehreren Bakterienstämme *Staphylococcus capitis* (*S. capitis*) N030_E12, *Staphylococcus epidermidis* (*S. epidermidis*) N009_G7, *S*. *epidermidis* N018_F3 oder eine beliebige Kombination davon umfassen, und
wobei mindestens einer des einen oder der mehreren Bakterienstämme in der Lage ist, ein Peptid zu exprimieren, das eine Sequenz umfasst, die aus der Gruppe ausgewählt ist, bestehend aus: MTKLAEAIANTVKAGQDHDWAKLGTSIVGIAENGIGLLGKVFGF (SEQ ID NO: 9437), MTKLAEAIANTVKAGQDHDWAKLGTSIVGIAENGIGALSKIFGG (SEQ ID NO: 9439), MQKLAEAIANTVKAGQDHDWAKLGTSIVGIAENGINAITKIFGG (SEQ ID NO: 9441), MTKLAEAIANAVKAGQDQDWAKLGTSIVGIAENGISLLGKVFGF (SEQ ID NO: 9443), MQKLAEAIANTVKAGQDHDWTKLGTSIVDIVENGVSALTKVFGG (SEQ ID NO: 9445) und MEKLFDAIRNTVDAGINQDWTKLGTSIVDIVDNGVKVISKFIGA (SEQ ID NO: 9447).

2. Topische probiotische Zusammensetzung zur Verwendung nach Anspruch 1, wobei die topische probiotische Zusammensetzung zur topischen Behandlung von Dysbiose-Funktionsstörungen der Haut, der Kopfhaut oder der Schleimhäute formuliert ist;
wobei die topische probiotische Zusammensetzung das Wachstum von *C. acnes* hemmt und
wobei der eine oder die mehreren Bakterienstämme in einer lebenden Form bereitgestellt werden.

3. Topische probiotische Zusammensetzung zur Verwendung nach einem der Ansprüche 1-2, wobei die topische probiotische Zusammensetzung zur topischen Behandlung von Dysbiose-Funktionsstörungen der Haut, der Kopfhaut oder der Schleimhäute formuliert ist;
wobei die topische probiotische Zusammensetzung das Wachstum von *C. acnes* hemmt und
wobei der eine oder die mehreren Bakterienstämme in einer lyophilisierten oder gefriergetrockneten oder sprühgetrockneten Form bereitgestellt werden.

4. Topische probiotische Zusammensetzung zur Verwendung nach Anspruch 3, wobei der eine oder die mehreren Bakterienstämme in eine lebende Form rekonstituiert werden können.

5. Topische probiotische Zusammensetzung zur Verwendung nach einem der Ansprüche 1-4, wobei die topische probiotische Zusammensetzung eine verdickte topische Formulierung des einen oder der mehreren Bakterienstämme umfasst;
wobei die topische probiotische Zusammensetzung zur topischen Behandlung von Dysbiose-Funktionsstörungen der Haut, der Kopfhaut oder der Schleimhäute formuliert ist;
wobei die topische probiotische Zusammensetzung das Wachstum von *C. acnes* hemmt und optional,
wobei die Zusammensetzung eine präbiotische Verbindung, ein Schutzmittel, ein Feuchthaltemittel, einen Weichmacher, ein Schleifmittel, ein Salz und/oder ein Tensid umfasst.

6. Topische probiotische Zusammensetzung zur Verwendung nach einem der Ansprüche 1-5, wobei die topische probiotische Zusammensetzung zur Behandlung von Dysbiose-Funktionsstörungen der Haut, der Kopfhaut oder der Schleimhäute formuliert ist und/oder wobei die Zusammensetzung als Creme, Salbe, Pomade, Spray, Pulver, Öl, verdickte Formulierung oder Umschlag formuliert ist.

7. Topische probiotische Zusammensetzung zur Verwendung nach einem der Ansprüche 1-6, wobei die Zusammensetzung das Wachstum, die Lebensfähigkeit oder die Aktivität von *C. acnes* hemmt.

8. Topische probiotische Zusammensetzung zur Verwendung nach einem der Ansprüche 1-7, wobei die dermatologische Erkrankung oder Funktionsstörung Akne, chronische Blepharitis und/oder Endophthalmitis ist.

9. Pharmazeutische Zusammensetzung, umfassend eine Vielzahl von phenollöslichen Modulin-Peptiden (PSM), umfassend PSMβ1 (SEQ ID NO: 9437), PSMβ3 (SEQ ID NO: 9441), PSMβ4 (SEQ ID NO: 9443) und PSMβ6 (SEQ ID NO: 9447); optional, wobei die Vielzahl von phenollöslichen Modulin-Peptiden (PSM) PSMβ1 (SEQ ID NO: 9437), PSMβ2 (SEQ ID NO: 9439), PSMβ3 (SEQ ID NO: 9441), PSMβ4 (SEQ ID NO: 9443), PSMβ5 (SEQ ID NO: 9445) und PSMβ6 (SEQ ID NO: 9447); und weiter optional einen pharmazeutisch verträglichen Träger umfasst.

10. Pharmazeutische Zusammensetzung nach Anspruch 9, wobei die pharmazeutische Zusammensetzung ein oder mehrere Bakterien umfasst, die aus der Gruppe bestehend aus *S. capitis* N030_E12, *S. epidermidis* N009_G7 und *S. epidermidis* N018_F3 ausgewählt sind.

11. Pharmazeutische Zusammensetzung nach einem der Ansprüche 9-10, umfassend einen zellfreien Fermentationsextrakt, erhalten aus einer Kultur von *S. capitis* N030_E12, *S. epidermidis* N009_G7, *S. epidermidis* N018_F3 oder einer beliebigen Kombination davon.

12. Pharmazeutische Zusammensetzung nach einem der Ansprüche 9-11 zur Verwendung bei der Behandlung einer dermatologischen Funktionsstörung im Zusammenhang mit *C. acnes,* Haut- oder Schleimhautinfektionen, atopischer Dermatitis, Psoriasis, Mastitis, Akne, chronischer Blepharitis und Endophthalmitis oder anderen Funktionsstörungen im Zusammenhang mit Hautdysbiose bei Menschen oder anderen Säugetieren, wobei die Zusammensetzung zur Anwendung auf die Haut oder Schleimhaut in einer wirksamen Menge der pharmazeutischen Zusammensetzung bei einem Patienten, der diese benötigt, dient.

13. Pharmazeutische Zusammensetzung nach Anspruch 12, wobei die pharmazeutische Zusammensetzung zur topischen Anwendung dient und wobei die pharmazeutische Zusammensetzung als Creme, Salbe, Pomade, Spray, Pulver, Öl, Lotion, Tinktur, verdickte Formulierung oder Umschlag formuliert ist.

14. Pharmazeutische Zusammensetzung nach einem der Ansprüche 12-13, wobei die pharmazeutische Zusammensetzung das Wachstum, die Lebensfähigkeit oder die Aktivität von *C. acnes* hemmt.

## Revendications

1. Composition probiotique topique comprenant une ou plusieurs souches bactériennes destinées à être utilisées dans le traitement d'une maladie ou d'un trouble dermatologique associé au *Cutibacterium acnes (C. acnes),* dans laquelle la ou les souches bactériennes comprennent le *Staphylococcus capitis (S. capitis)* N030_E12, le *Staphylococcus epidermidis (S. epidermidis)* N009_G7, *S. epidermidis* N018_F3, ou toute combinaison de ceux-ci, et
dans laquelle au moins l'une de la ou des souches bactériennes est capable d'exprimer un peptide comprenant une séquence sélectionnée parmi le groupe consistant en : MTKLAEAIANTVKAGQDHDWAKLGTSIVGIAENGIGLLGKVFGF (SEQ ID NO: 9437), MTKLAEAIANTVKAGQDHDWAKLGTSIVGIAENGIGALSKIFGG (SEQ ID NO: 9439), MQKLAEAIANTVKAGQDHDWAKLGTSIVGIAENGINAITKIFGG (SEQ ID NO: 9441), MTKLAEAIANAVKAGQDQDWAKLGTSIVGIAENGISLLGKVFGF (SEQ ID NO: 9443), MQKLAEAIANTVKAGQDHDWTKLGTSIVDIVENGVSALTKVFGG (SEQ ID NO: 9445), et MEKLFDAIRNTVDAGINQDWTKLGTSIVDIVDNGVKVISKFIGA (SEQ ID NO: 9447).

2. Composition probiotique topique destinée à être utilisée selon la revendication 1, dans laquelle la composition probiotique topique est formulée pour le traitement topique des troubles de la dysbiose de la peau, du cuir chevelu ou des muqueuses ;
dans laquelle la composition probiotique topique inhibe la croissance du C. *acnes'*
et
dans laquelle la ou les souches bactériennes sont fournies sous forme vivante.

3. Composition probiotique topique destinée à être utilisée selon l'une quelconque des revendications 1 à 2, dans laquelle la composition probiotique topique est formulée pour le traitement topique des troubles de la dysbiose de la peau, du cuir chevelu ou des muqueuses ;
dans laquelle la composition probiotique topique inhibe la croissance du C. *acnes,*
et
dans laquelle la ou les souches bactériennes sont fournies sous forme lyophilisée, cryodesséchée ou desséchée par atomisation.

4. Composition probiotique topique destinée à être utilisée selon la revendication 3, dans lequel la ou les souches bactériennes peuvent être reconstituées sous une forme vivante.

5. Composition probiotique topique destinée à être utilisée selon l'une quelconque des revendications 1 à 4, dans laquelle la composition probiotique topique comprend une formulation topique épaissie de la ou des souches bactériennes ;
dans laquelle la composition probiotique topique est formulée pour le traitement topique des troubles de la dysbiose de la peau, du cuir chevelu ou des muqueuses ;
dans laquelle la composition probiotique topique inhibe la croissance du C. *acnes,* et éventuellement,
dans laquelle la composition comprend un composé prébiotique, un protecteur, un humectant, un émollient, un abrasif, un sel et/ou un agent tensioactif.

6. Composition probiotique topique destinée à être utilisée selon l'une quelconque des revendications 1 à 5, dans laquelle la composition probiotique topique est formulée pour le traitement des troubles de la dysbiose de la peau, du cuir chevelu ou des muqueuses, et/ou dans laquelle la composition est formulée sous forme de crème, de pommade, d'onguent, de spray, de poudre, d'huile, de formulation épaissie, ou de cataplasme.

7. Composition probiotique topique destinée à être utilisée selon l'une quelconque des revendications 1 à 6, dans laquelle la composition inhibe la croissance, la viabilité ou l'activité du C. *acnes.*

8. Composition probiotique topique destinée à être utilisée selon l'une quelconque des revendications 1 à 7, dans laquelle la maladie ou le trouble dermatologique est l'acné, la blépharite chronique et/ou l'endophtalmie.

9. Composition pharmaceutique comprenant une pluralité de peptides de moduline solubles dans le phénol (PSM), comprenant PSMβ1 (SEQ ID NO : 9437), PSMβ3 (SEQ ID NO : 9441), PSMβ4 (SEQ ID NO : 9443), et PSMβ6 (SEQ ID NO : 9447) ; éventuellement, la pluralité de peptides de moduline solubles dans le phénol (PSM) comprend PSMβ1 (SEQ ID NO : 9437), PSMβ2 (SEQ ID NO : 9439), PSMβ3 (SEQ ID NO : 9441), PSMβ4 (SEQ ID NO : 9443), PSMβ5 (SEQ ID NO : 9445) et PSMβ6 (SEQ ID NO : 9447) ; et, éventuellement, un support pharmaceutiquement acceptable.

10. Composition pharmaceutique selon la revendication 9, dans laquelle la composition pharmaceutique comprend une ou plusieurs bactéries sélectionnées parmi le groupe consistant en *S. capitis* N030_E12, *S. epidermidis* N009_G7, et S. *epidermidis* N018_F3.

11. Composition pharmaceutique selon l'une quelconque des revendications 9 à 10, comprenant un extrait de fermentation acellulaire obtenu à partir d'une culture de S. *capitis* N030_E12, *S. epidermidis* N009_G7, *S*. *epidermidis* N018_F3, ou toute combinaison de ceux-ci.

12. Composition pharmaceutique selon l'une quelconque des revendications 9 à 11 destinée à être utilisée dans le traitement d'un trouble dermatologique associé au C. *acnes,* les infections cutanées ou des muqueuses, la dermatite atopique, le psoriasis, la mastite, l'acné, la blépharite chronique et l'endophtalmie, ou d'autres troubles liés à la dysbiose cutanée chez l'homme ou d'autres mammifères, dans laquelle la composition est destinée à être appliquée sur la peau ou les muqueuses dans une quantité efficace de la composition pharmaceutique chez un sujet qui en a besoin.

13. Composition pharmaceutique destinée à être utilisée selon la revendication 12, dans laquelle la composition pharmaceutique est destinée à une application topique, et dans laquelle la composition pharmaceutique est formulée sous forme de crème, de pommade, d'onguent, de spray, de poudre, d'huile, de lotion, de teinture, de formulation épaissie, ou de cataplasme.

14. Composition pharmaceutique destinée à être utilisée selon l'une quelconque des revendications 12 à 13, dans laquelle la composition pharmaceutique inhibe la croissance, la viabilité ou l'activité du C. *acnes.*
